(19) Europäisches Patentamt
European Patent Office
Office européen des brevets

(11) EP 1 041 953 B1

# (12) EUROPÄISCHE PATENTSCHRIFT

(45) Veröffentlichungstag und Bekanntmachung des Hinweises auf die Patenterteilung:
**07.07.2004 Patentblatt 2004/28**

(21) Anmeldenummer: **99911709.6**

(22) Anmeldetag: **26.02.1999**

(51) Int Cl.[7]: **A61K 7/00**

(86) Internationale Anmeldenummer:
**PCT/EP1999/001236**

(87) Internationale Veröffentlichungsnummer:
**WO 1999/059528 (25.11.1999 Gazette 1999/47)**

(54) **MITTEL UND VERFAHREN ZUR ERZEUGUNG VON TEMPORÄREN FÄRBUNGEN AUF KERATINFASERN**

AGENTS AND METHOD FOR PRODUCING TEMPORARY COLORATIONS OF KERATIN FIBERS

PRODUITS ET PROCEDE DESTINES A DES COLORATIONS TEMPORAIRES DE FIBRES KERATINIQUES

(84) Benannte Vertragsstaaten:
**AT CH DE ES FR GB IT LI**

(30) Priorität: **16.05.1998 DE 19822199**

(43) Veröffentlichungstag der Anmeldung:
**11.10.2000 Patentblatt 2000/41**

(73) Patentinhaber: **Wella Aktiengesellschaft
64274 Darmstadt (DE)**

(72) Erfinder:
- **GÖTTEL, Otto
CH-1723 Marly (CH)**
- **PIRRELLO, Aline
CH-1762 Givisiez (CH)**
- **METTLER, Sandra
CH-1753 Matran (CH)**

(56) Entgegenhaltungen:
**EP-A- 0 833 314**

- **BERLIN ET AL.: "Methoden zur Herstellung von Cyaninen (Polymethinen)" HOUBEN-WEYL, Bd. 5, Nr. 1D, Seiten 227-299, XP002117851**
- **HENSLEY ET AL.: "POLYMETHINE DYES" KIRK-OTHMER ENCYCL. CHEM. TECHNOL. SECOND EDITION, Bd. 16, 1968, Seiten 282-291, XP002117852**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 041 953 B1

## Beschreibung

Die vorliegende Erfindung betrifft nicht-oxidative Färbemittel für Keratinfasem, wie zum Beispiel Haare oder Wolle, auf der Basis von Polymethinfarbstoffen sowie ein Verfahren zum temporären Färben von Keratinfasern, bei dem die Färbung zu einem beliebigen Zeitpunkt wieder entfernt werden kann.

Die Färbung von Haaren ist in der heutigen Zeit den unterschiedlichsten Trends unterworfen. Während früher Haare vor allem gefärbt wurden, um Grauanteile zu überdecken, besteht heute immer mehr das Verlangen nach der Integration der Haarfarbe in die aktuelle Mode sowie als Ausdruck der Persönlichkeit.

Zur Färbung von Haaren stehen nach wie vor zwei etablierte Methoden zur Verfügung. Zum einen ist dies die oxidative Haarfärbung, die a priori für temporäre Färbungen ungeeignet ist, da sie zu einem sehr dauerhaften Färbeergebnis führt. Zum anderen besteht die Möglichkeit die Haare mit nicht-oxidativen, direktziehende Farbstoffe enthaltenden Färbemitteln (häufig als Tönungen bezeichnet) zu färben. Obwohl die dafür verwendeten Farbstoffe neben der färberischen Leistung darauf optimiert sind, möglichst lange im Haar zu verbleiben, werden mit jedem Waschvorgang die Tönungsergebnisse graduell schwächer, so daß je nach verwendetem Produkt und Haartyp solche Färbungen in der Regel maximal 10 Haarwäschen überdauem. Falls jedoch ein Benutzer dieser nicht-oxidativen Färbemittel zu einem früheren Zeitpunkt seine ursprüngliche Haarfarbe zurückerhalten möchte, gibt es bisher keine geeigneten Mittel, die die schnelle Wiederherstellung des ursprünglichen Farbtons in zufriedenstellender Weise erlauben, da die dazu eingesetzten Produkte in der Regel sehr aggressiv sind und Haarschädigungen zur Folge haben.

In der Literatur wird eine Vielzahl von Versuchen beschrieben, eine Färbung von Fasern wieder rückgängig zu machen. So wird in der DE-PS 38 42 774 und der US-PS 4 681 471 die Entfärbung von Triarylmethanfarbstoffen mit Reduktionsmitteln beschrieben. In die gleiche Richtung geht auch die US-PS 5 474 578, bei der zur Entfärbung der verwendeten Farbstoffe entweder eine oxidative oder eine reduktive Entfärbung oder aber eine Kombination von beiden Behandlungen verwendet wird. Ein generelles Problem bei diesen Methoden liegt insbesondere darin, daß die Entfärbung meist nur teilweise erfolgt. So ermöglicht das Verfahren nach der US-PS 5 474 578 im günstigsten Fall einen Entfärbegrad von maximal 90 bis 93%, wobei sich dieser Entfärbegrad nur bei einer aufeinanderfolgenden oxidativen und reduktiven Behandlung erzielen läßt. Diese doppelte Behandlung ist jedoch außerordentlich haarschädigend. Normalerweise wird bei diesem Verfahren lediglich eine Teilentfärbung (häufig < 50%) der Haare erzielt.

Den zuletzt genannten Patentanmeldungen ist weiterhin gemeinsam, daß sie sich mit der Entfärbung von in der Haarkosmetik alteingeführten Farbstoffklassen befassen. Die verwendeten Färbemittel basieren auf Direktziehern mit unterschiedlichen chemischen und physikalisch Eigenschaften, welche auch ein unterschiedliches Färbeverhalten und Bleichverhalten besitzen, wodurch spätestens bei auf Farbstoffgemischen aufbauenden Nuancierungen, eine gleichmäßige Entfärbung äußerst problematisch wird, da das Ergebnis von den Eigenschaften der leistungsschwächsten Komponente bestimmt wird.

Es bestand daher ein Bedürfnis nach nicht-oxidativen Färbemitteln, welche jederzeit ohne große Schädigung der Keratinfaser wieder entfernt werden können.

Überraschenderweise wurde nunmehr gefunden, daß Färbemittel auf der Basis von anionischen oder neutralen Mono- bzw. Polymethinfarbstoffen eine hervorragende Färbung von Keratinfasern ermöglichen, wobei sich die erhaltenen Färbungen auf einfache Weise innerhalb kurzer Zeit wieder entfernen lassen. Zur Entfärbung sind hierbei sowohl reduzierende als auch oxidierende Mittel geeignet.

Farbstoffe dieses Typs sind bereits seit langem bekannt und man findet in der Literatur eine sehr breite Palette von Substitutionsmustern. Einen Überblick über diese Farbstoffe und die Herstellungsmethoden findet man beispielsweise in der JP-OS 03-204640. Verschiedene Farbstoffe sind zudem im Handel erhältlich.

Mit den erfindungsgemäßen Färbemitteln lassen sich Nuancen im abgewandelten Naturton, insbesondere aber im modischen Bereich erzielen. Darüber hinaus ist es möglich, eine Reihe von brillanten Farbreflexen vor allem im gelben bis rotvioletten Bereich zu erzielen. Bedingt durch die sehr hohe Farbstärke der Farbstoffe und das gute Aufziehverhalten kann in hervorragender Weise der ursprüngliche Farbton der Faser überdeckt werden. Damit kann auch dem eingangs erwähnten Wunsch nach der Integration der Haarfarbe in die Mode sowie als Ausdruck der Persönlichkeit voll entsprochen werden.

Gegenstand der vorliegenden Anmeldung ist daher ein Mittel zur Färbung von Keratinfasem, wie zum Beispiel Pelzen, Federn, Wolle oder Haaren, insbesondere menschlichen Haaren, welches dadurch gekennzeichnet ist, daß es mindestens einen Pyrazolonfarbstoff der allgemeinen Struktur (I) oder dessen physiologisch verträgliches Salz enthält,

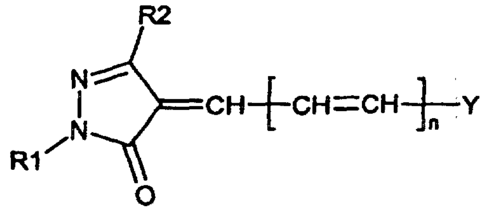

(I)

worin **R1** Wasserstoff, eine geradkettige oder verzweigte C1- bis C8-Alkylgruppe, eine Hydroxyethylgruppe, eine Dihydroxypropylgruppe, eine Methoxyethylgruppe, eine Carboxyethylgruppe, eine C1- bis C4-Sulfoalkylgruppe, einen Phenylrest, einen mit einem oder mehreren Halogenatomen substituierten Phenylrest, einen mit einer oder zwei Sulfonsäuregruppen substituierten Phenylrest, einen mit einer oder zwei Carbonsäuregruppen substituierten Phenylrest, einen mit einer oder mehreren unverzweigten oder verzweigten C1- bis C8-Alkylgruppen substituierten Phenylrest, einen mit einer oder mehreren unverzweigten oder verzweigten C1- bis C8-Alkoxygruppen substituierten Phenylrest, einen Benzylrest, einen mit einem oder mehreren Halogenatomen substituierten Benzylrest, einen mit einer C1- bis C4-Alkylgruppe substituierten Benzylrest, einen mit einer Hydroxygruppe substituierten Benzylrest, einen mit einer Methoxygruppe substituierten Benzylrest, einen mit einer Carboxygruppe substituierten Benzylrest, einen mit einer Nitrogruppe substituierten Benzylrest, einen mit einer Aminogruppe substituierten Benzylrest oder einen fünfgliedrigen oder sechsgliedrigen gesättigten oder ungesättigten Heterocyclus bedeutet; R2 Wasserstoff, eine verzweigte oder unverzweigte C1- bis C6-Alkylgruppe, einen Phenylrest, eine Aminogruppe, eine acylierte oder sulfonylierte Aminogruppe, eine Acetylgruppe, eine Methoxygruppe, eine Carbonsäuregruppe, eine mit einem geradkettigen oder verzweigten C1-bis C8-Alkohol oder einem Ethylenglykolmonomethylether oder einem Ethylenglykolmonoethylether veresterte Carbonsäuregruppe, eine Carbonsäureamidgruppe, eine Carbonsäureanilidgruppe oder eine 2-Amino-2-oxyethylgruppe bedeutet;
Y einen aromatischen fünfgliedrigen oder sechsgliedrigen carbocyclischen oder heterocyclischen Ring oder einen benzoanellierten aromatischen fünfgliedrigen oder sechsgliedrigen carbocyclischen oder heterocyclischen Ring darstellt, oder Y für einen Rest der allgemeinen Formel (II) oder (III),

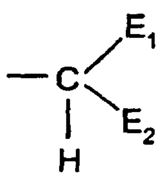

(II),

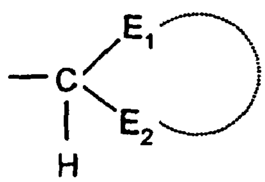

(III),

in der **E1** und **E2** unabhängig voneinander eine Nitrilgruppe, eine Alkylsulfonylgruppe, eine Acylgruppe, eine Carbonestergruppe oder eine Carbonamidgruppe bedeuten, steht; und n gleich 0, 1 oder 2 ist.

Als physiologisch verträgliche Salze der Verbindungen der Formel (I) sind insbesondere Pyridiniumsalze, Tetraalkylphosphoniumsalze, Tetraarylphosphoniumsalze, Alkalisalze und Ammoniumsalze, beispielsweise die Ammoniumsalze, Triethylammoniumsalze, Natriumsalze, Kaliumsalze, N-Methylmorpholiniumsalze, Monoethanolammoniumsalze, Diethanolammoniumsalze und Triethanolammoniumsalze, zu nennen, wobei die Natriumsalze, Kaliumsalze und insbesondere die Ammoniumsalze bevorzugt sind.

Besonders bevorzugt sind Pyrazolonfarbstoffe der allgemeinen Struktur (I), worin
**R1** eine geradkettige oder verzweigte C1- bis C4- Alkylgruppe, eine Hydroxyethylgruppe, eine Dihydroxypropylgruppe, eine Methoxyethylgruppe, eine C1- bis C4-Sulfoalkylgruppe, einen Phenylrest, einen mit einem oder mehreren Halogenatomen substituierten Phenylrest, einen mit einer oder zwei Sulfonsäuregruppen substituierten Phenylrest oder einen mit einer oder zwei Carbonsäuregruppen substituierten Phenylrest bedeutet;
**R2** Wasserstoff, eine Methylgruppe, eine Carbonsäuregruppe, eine mit einem geradkettigen C1- bis C4-Alkohol veresterte Carbonsäuregruppe oder eine Carbonsäureamidgruppe bedeutet;
Y einen Donor-substituierten Phenylrest, einen aromatischen fünfgliedrigen heterocyclischen Ring oder einen benzoanellierten aromatischen fünfgliedrigen heterocyclischen Ring darstellt; und
n gleich 0, 1 oder 2 ist.

Beispiele für geeignete Verbindungen der Formel (I) sind (jeweils in der Säureform geschrieben):

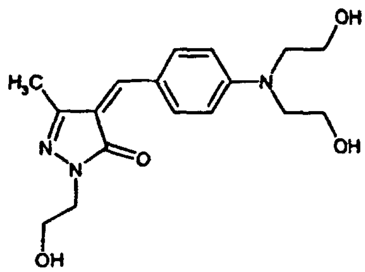

4-(4-(Bis-(2-hydroxyethyl)amino)-benzyliden)-2-(2-hydroxyethyl)-5-methyl-2,4-dihydro-pyrazol-3-on **(1)**

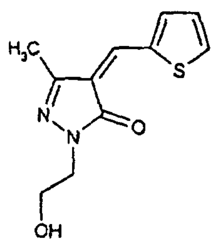

2-(2-Hydroxyethyl)-5-methyl-4-thiophen-2-ylmethylen-2,4-dihydro-pyrazol-3-on **(2)**

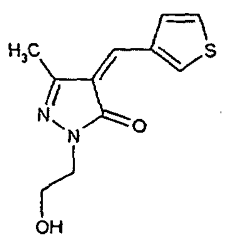

2-(2-hydroxyethyl)-5-methyl-4-thiophen-3-ylmethylen-2,4-dihydro-pyrazol-3-on (3)

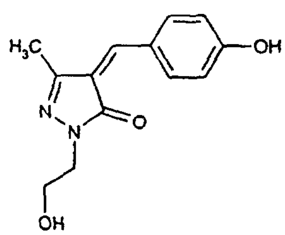

4-(4-Hydroxy-benzyliden)-2-(2-hydroxyethyl)-5-methyl-2,4-dihydro-pyrazol-3-on (4)

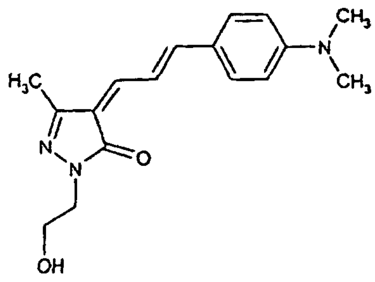

4-(3-(4-Dimethylamino-phenyl)-allyliden)-2-(2-hydroxyethyl)-5-methyl-2,4-dihydro-pyrazol-3-on **(5)**

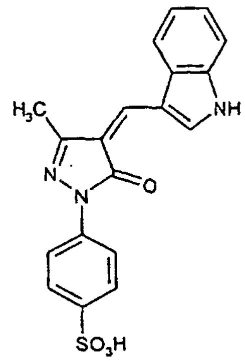

4-(4-(1H-Indol-3-ylmethylen)-3-methyl-5-oxo-4,5-dihydro-pyrazol-1-yl)-benzolsulfonsäure **(6)**

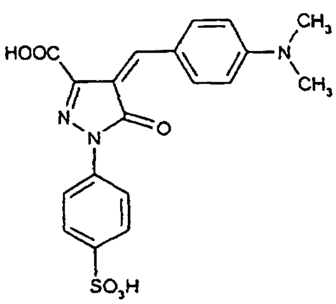

4-(4-Dimethylamino-benzyliden)-5-oxo-1-(4-sulfo-phenyl)-4,5-dihydro-1Hpyrazol-3-carbonsäure **(7)**

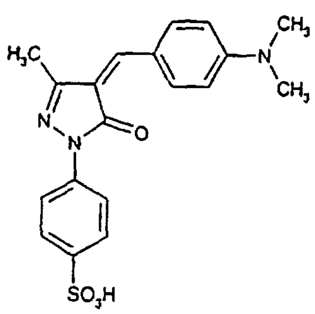

4-(4-(4-Dimethylamino-benzyliden)-3-methyl-5-oxo-4,5-dihydro-pyrazol-1-yl)-benzolsulfonsäure **(8)**

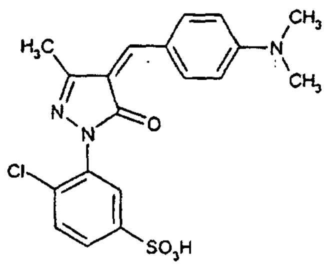

4-Chlor-3-(4-(4-dimethylamino-benzyliden)-3-methyl-5-oxo-4,5-dihydropyrazol-1-yl)-benzolsulfonsäure **(9)**

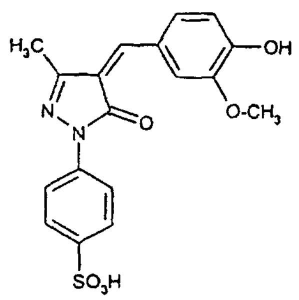

4-(4-(4-Hydroxy-3-methoxy-benzyliden)-3-methyl-5-oxo-4,5-dihydropyrazol-1-yl)-benzolsulfonsäure **(10)**

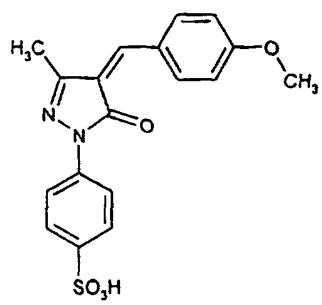

4-(4-(4-Methoxy-benzyliden)-3-methyl-5-oxo-4,5-dihydro-pyrazol-1 -yl)-benzolsulfonsäure **(11)**

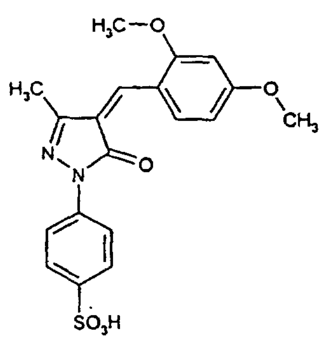

4-(4-(2,4-Dimethoxy-benzyliden)-3-methyl-5-oxo-4,5-dihydro-pyrazol-1-yl)-benzolsulfonsäure **(12)**

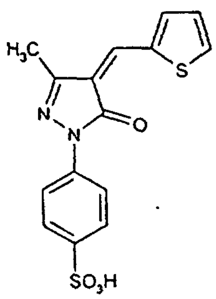

4-(3-Methyl-5-oxo-4-thiophen-2-ylmethylen-4,5-dihydro-pyrazol-1-yl)-benzolsulfonsäure **(13)**

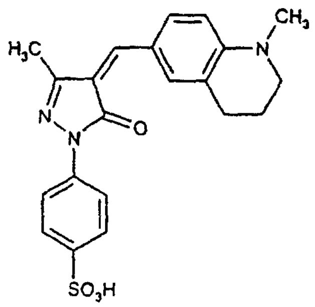

4-(3-Methyl-4-(1-methyl-1,2,3,4-tetrahydro-chinolin-6-ylmethylen)-5-oxo-4,5-dihydro-pyrazol-1-yl)-benzolsulfonsäure **(14)**

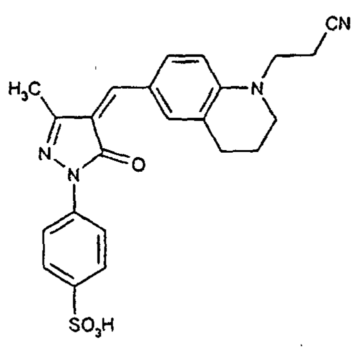

4-(4-(1-(2-Cyan-ethyl)-1,2,3,4-tetrahydro-chinolin-6-ylmethylen)-3-methyl-5-oxo-4,5-dihydro-pyrazol-1-yl)-benzolsulfonsäure **(15)**

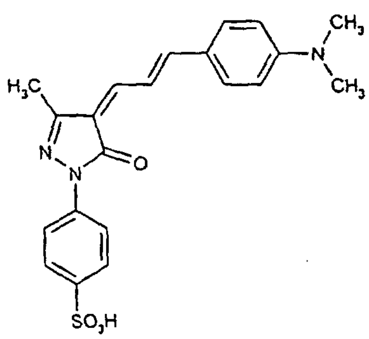

4-(4-(3-(4-Dimethylamino-phenyl)-allyliden)-3-methyl-5-oxo-4,5-dihydropyrazol-1-yl)-benzolsulfonsäure **(16)**

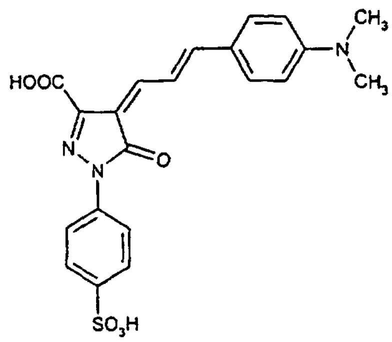

4-(3-(4-Dimethylamino-phenyl)-allyliden)-5-oxo-1-(4-sulfophenyl)-4,5-dihydro-1H-pyrazol-3-carbonsäure **(17)**

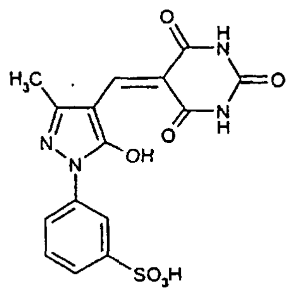

3-(5-Hydroxy-3-methyl-4-(2,4,6-trioxo-tetrahydropyrimidin-5-ylidenmethyl)-pyrazol-1-yl)-benzolsulfonsäure **(18)**

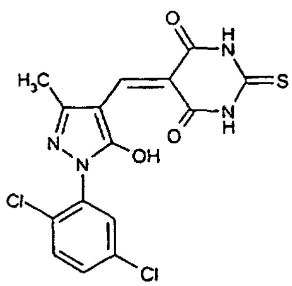

5-(1-(2,5-Dichlorphenyl)-5-hydroxy-3-methyl-1H-pyrazol-4-ylmethylen)-2-thioxo-dihydro-pyrimidin-4,6-dion **(19)**

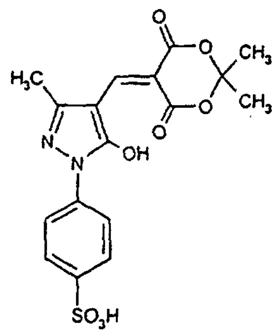

4-(4-(2,2-Dimethyl-4,6-dioxo-[1,3]dioxan-5-ylidenmethyl)-5-hydroxy-3-methyl-pyrazol-1-yl)-benzolsulfonsäure **(20)**

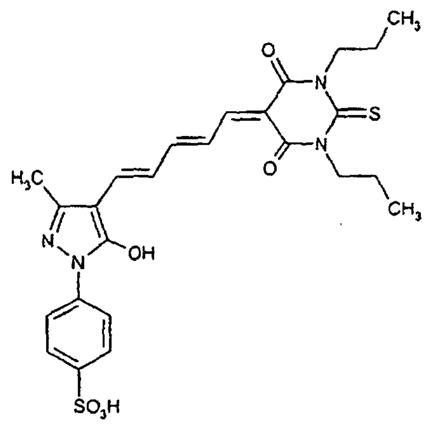

4-(4-(5-(4,6-Dioxo-1,3-dipropyl-2-thioxo-tetrahydro-pyrimidin-5-yliden)-penta-1,3-dienyl)-5-hydroxy-3-methyl-pyrazol-1-yl)-benzolsulfonsäure **(21)**

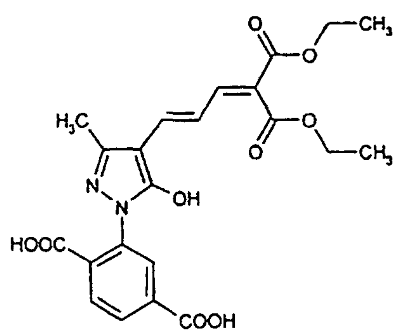

2-(4-(4,4-Bis-ethoxycarbonyl-buta-1,3-dienyl)-5-hydroxy-3-methyl-pyrazol-1-yl)-terephthalsäure **(22)**

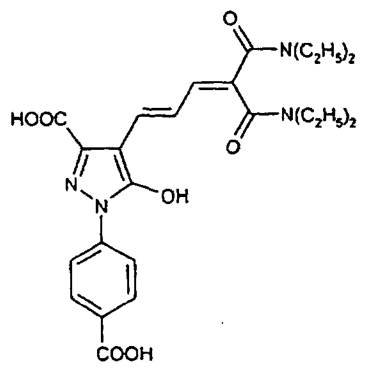

1-(4-Carboxy-phenyl)-4-(4,4-dicarbamoyl-buta-1,3-dienyl)-5-hydroxy-1Hpyrazol-3-carbonsäure **(23)**

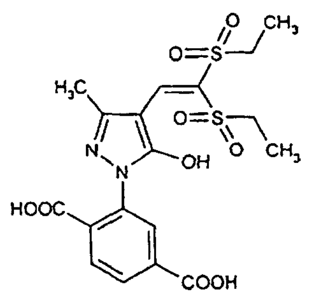

2-(4-(2,2-Bis-ethansulfonyl-vinyl)-5-hydroxy-3-methyl-pyrazol-1-yl)-terephthalsäure **(24)**

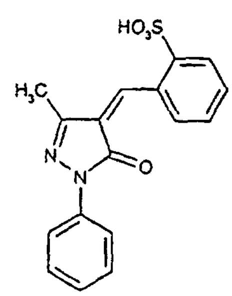

2-(3-Methyl-5-oxo-1-phenyl-1,5-dihydro-pyrazol-4-ylidenemethyl)-benzolsulfonsäure **(25)**

Besonders geeignet sind Farbstoffe der Formel (I) oder (IV), die Sulfonsäuregruppen enthalten und in Form ihrer Natriumsalze, Kaliumsalze, Ammoniumsalze oder Triethylammoniumsalze vorliegen, wie zum Beispiel:

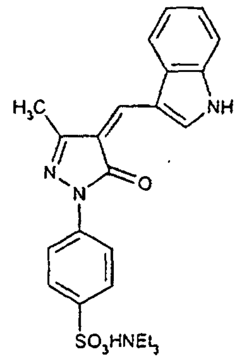

Triethylammonium-4-(4-(1H-indol-3-ylmethylen)-3-methyl-5-oxo-4,5-dihydro-pyrazol-1-yl)-benzolsulfonat **(6a)**

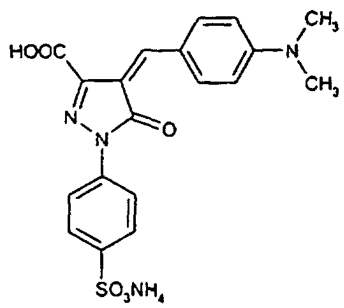

Ammonium-4-(4-dimethylamino-benzyliden)-5-oxo-1-(4-sulfo-phenyl)-4,5-dihydro-1H-pyrazol-3-carbonsäure **(7a)**

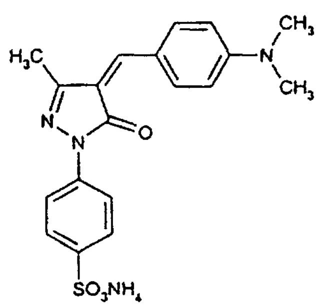

Ammonium-4-(4-(4-dimethylamino-benzyliden)-3-methyl-5-oxo-4,5-dihydro-pyrazol-1-yl)-benzolsulfonat **(8a)**

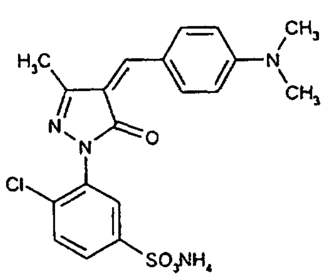

Ammonium-4-chlor-3-(4-(4-dimethylamino-benzyliden)-3-methyl-5-oxo-4,5-dihydro-pyrazol-1-yl)-benzolsulfonat **(9a)**

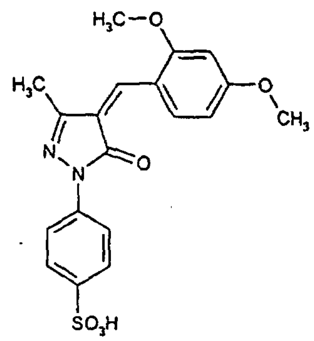

Ammonium-4-(4-(2,4-dimethoxy-benzyliden)-3-methyl-5-oxo-4,5-dihydropyrazol-1-yl)-benzolsulfonat **(12a)**

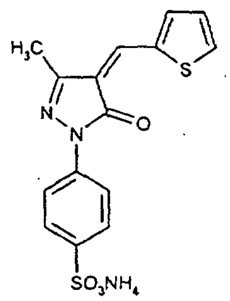

Ammonium-4-(3-methyl-5-oxo-4-thiophen-2-ylmethylen-4,5-dihydropyrazol-1-yl)-benzolsulfonsäure **(13a)**

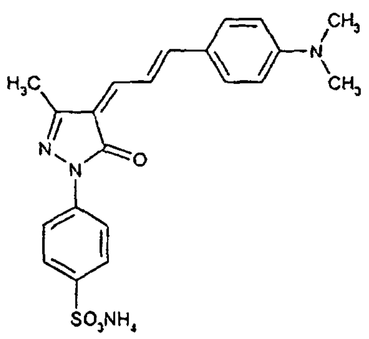

Ammonium-4-(4-(3-(4-dimethylamino-phenyl)-allyliden)-3-methyl-5-oxo-4,5-dihydro-pyrazol-1-yl)-benzolsulfonat **(16a)**

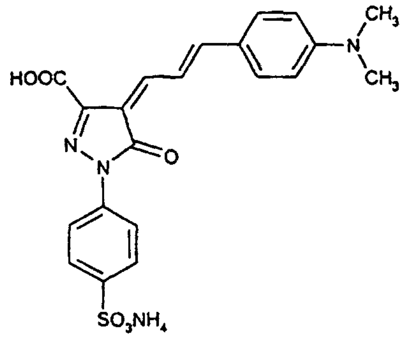

Ammonium-4-(3-(4-dimethylamino-phenyl)-allyliden)-5-oxo-1-(4-sulfophenyl)-4,5-dihydro-1H-pyrazol-3-carbonsäure **(17a)**

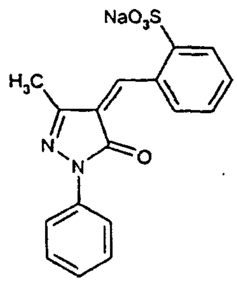

Natrium-2-(3-methyl-5-oxo-1-phenyl-1,5-dihydro-pyrazol-4-ylidenemethyl)-benzolsulfonat **(23a)**

Kombinationen aus den vorgenannten Farbstoffen der Formel (I) ermöglichen eine vom Haaransatz bis zur Haarspitze gleichmäßige Färbung im leicht abgewandelten Naturtonbereich, vor allem jedoch bei leuchtend modischen Färbungen mit orangeroten Reflexen. Die vorzüglichen Eigenschaften der neuen Farbstoffe zeigen sich besonders auf durch Licht und Wetter geschädigtem oder auf dauergewellten Haaren.

Obwohl viele der bevorzugten Farbstoffe der allgemeinen Struktur (I) einen anionischen Charakter aufweisen und ein relativ hohes Molekulargewicht besitzen (was in der Haarkosmetik normalerweise nachteilig ist), ziehen sie überraschend gut auf das Haar auf.

Der Gesamtgehalt an den Pyrazolonfarbstoffen der Formel (I) beträgt in dem erfindungsgemäßen Haarfärbemittel etwa 0,01 bis 5 Gewichtsprozent, vorzugsweise 0,5 bis 4 Gewichtsprozent.

Zur Erhöhung der Farbintensität können die in kosmetischen Systemen üblichen Carrier zugesetzt werden. Geeignete Verbindungen sind beispielsweise Benzylalkohol, Vanillin oder Isovanillin. Weitere geeignete Carrier werden in der DE-OS 196 18 595 beschrieben, auf die hiermit ausdrücklich Bezug genommen wird.

Das erfindungsgemäße Mittel zur Färbung von Keratinfasem kann beispielsweise in Form einer Lösung, insbesondere als wäßrigalkoholische Lösung, einer Creme, eines Geles oder einer Emulsion. vorliegen. Als Lösungsmittel können neben Wasser beispielsweise niedere aliphatische einwertige oder mehrwertige Alkohole, deren Ester und Ether, oder aber Gemische dieser Lösungsmittel untereinander oder mit Wasser genannt werden. Der maximale Siedepunkt der vorgenannten geeigneten Lösungsmittel beträgt etwa 400°C, wobei ein Siedepunkt von 20°C bis 250°C bevorzugt ist.

Ebenfalls ist es möglich, dieses Mittel mit Hilfe eines Zerstäubers beziehungsweise anderer geeigneter Pumpvorrichtungen oder Sprühvorrichtungen oder im Gemisch mit üblichen unter Druck verflüssigten Treibmitteln als Aerosolspray oder als Aerosolschaum aus einem Druckbehälter zu entnehmen.

Der pH-Wert des erfindungsgemäßen Färbemittels beträgt 2 bis 11, wobei ein pH-Wert von 2,5 bis 8 besonders bevorzugt ist. Die Einstellung eines alkalischen pH-Wertes erfolgt vorzugsweise mit Ammoniak, es ist jedoch auch möglich, anstelle von Ammoniak organische Amine, wie zum Beispiel Monoethanolamin oder Triethanolamin zu verwenden. Für die Einstellung eines sauren pH-Wertes kann hingegen eine organische oder anorganische Säure, wie zum Beispiel Salzsäure, Schwefelsäure, Phosphorsäure, Ascorbinsäure, Glycolsäure oder Milchsäure, verwendet werden.

Selbstverständlich kann das vorstehend beschriebene Färbemittel gegebenenfalls weitere für Färbemittel für Keratinfasern übliche Zusätze, wie zum Beispiel Pflegestoffe, Netzmittel, Verdicker, Weichmacher, Konservierungsstoffe und Parfümöle sowie auch weitere, nachstehend aufgeführte Zusätze enthalten.

Weiterhin können in dem erfindungsgemäßen Färbemittel Netzmittel oder Emulgatoren aus den Klassen der anionischen, amphoteren, nichtionogenen oder zwitterionischen oberflächenaktiven Substanzen wie Fettalkoholsulfate, Alkansulfonate, Alkylbenzolsulfonate, Alkylbetaine, α-Olefinsulfonate, oxethylierte Fettalkohole, oxethylierte Nonylphenole, Fettsäurealkanolamine, oxethylierte Fettsäureester, Fettalkoholpolyglycolethersulfate, Alkylpolyglucoside, Verdickungsmittel wie höhere Fettalkohole, Stärke, Alginate, Bentonite, Cellulosederivate, Vaseline, Paraffinöl und Fettsäuren, wasserlösliche polymere Verdickungsmittel wie natürliche Gummiarten, Guargummi, Xanthangummi, Johannisbrotkemmehl, Pektin, Dextran, Agar-Agar, Amylose, Amylopektin, Dextrine, Tone oder vollsynthetische Hydrokolloide wie Polyvinylalkohol, außerdem Pflegestoffe wie Lanolinderivate, Cholesterin, Pantothensäure, wasserlösliche Polymere, Proteinderivate, Provitamine, Vitamine, Pflanzenextrakte, Zucker und Betain, Hilfsstoffe wie Feuchthaltemittel, Elektrolyte, Antioxidantien, Fettamide, Sequestrierungsmittel, filmbildende Agentien und Konservierungsmittel, enthalten sein.

Das vorstehend beschriebene Färbemittel kann weiterhin natürliche oder synthetische Polymere beziehungsweise modifizierte Polymere natürlichen Ursprungs enthalten sein, wodurch gleichzeitig mit der Färbung eine Festigung der Keratinfaser erreicht wird. Solche Mittel werden im allgemeinen als Tönungsfestiger oder Farbfestiger bezeichnet. Von den für diesen Zweck in der Kosmetik bekannten synthetischen Polymeren seien beispielsweise Polyvinylpyrrolidon, Polyvinylacetat, Polyvinylalkohol oder Polyacrylverbindungen wie Polyacrylsäure oder Polymethacrylsäure, Polyacrylnitril, Polyvinylacetate sowie Copolymerisate aus derartigen Verbindungen, wie zum Beispiel Polyvinylpyrrolidon-Vinylacetat, erwähnt; während als natürliche Polymere oder modifizierte natürliche Polymere beispielsweise Chitosan (deacetyliertes Chitin) oder Chitosanderivate, eingesetzt werden können.

Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Menge von etwa 0,1 bis 5 Gewichtsprozent. Die vorgenannten Polymere können in dem erfindungsgemäßen Mittel in der für solche Mittel üblichen Menge, insbesondere in einer Menge von etwa 1 bis 5 Gewichtsprozent, verwendet werden.

Das erfindungsgemäße Mittel zur Färbung von Keratinfasem eignet sich insbesondere zu Färbung von Haaren. Das erfindungsgemäße Färbemittel wird hierzu in üblicher Weise in einer für die Färbung der Haare ausreichende Menge, im allgemeinen etwa 50 bis 150 Gramm, auf das Haar aufgetragen. Nach einer für die Haarfärbung ausreichenden Einwirkungszeit, die üblicherweise etwa 10 bis 45 Minuten bei 20 bis 50 °C, vorzugsweise 15 bis 30 Minuten bei etwa 40°C, beträgt, wird das Haar mit Wasser gespült, gegebenenfalls mit einem Shampoo gewaschen und/oder mit der wäßrigen Lösung einer schwachen organischen Säure, wie zum Beispiel Zitronensäure oder Weinsäure, nachgespült und getrocknet.

Die Anwendung des Färbemittels mit zusätzlicher Festigung erfolgt in bekannter und üblicher Weise durch Befeuchten des Haares mit dem Festiger, Einlegen des Haares zur Frisur und anschließende Trocknung.

Hinsichtlich der färberischen Möglichkeiten bietet das erfindungsgemäße Haarfärbemittel je nach Art und Zusammensetzung der verwendeten Pyrazolonfarbstoffe eine breite Palette verschiedener Farbnuancen, die sich von natürlichen Farbtönen bis hin zu hochmodischen, leuchtenden Nuancen erstreckt. Die vorzüglichen Eigenschaften der neuen Färbemittel zeigen sich insbesondere auf durch Licht und Wetter geschädigtem oder auf dauergewellten Haaren.

Obwohl in dem erfindungsgemäßen Färbemittel zusätzlich auch natürliche oder synthetische direktziehende Farbstoffe, beispielsweise Nitrofarbstoffe, Azofarbstoffe, Chinonfarbstoffe, Triphenylmethanfarbstoffe oder basische beziehungsweise saure Farbstoffe, enthalten sein können, werden vorzugsweise ausschließlich Pyrazolonfarbstoffe der Formel (I), welche als strukturanaloge Verbindungen alle zueinander kompatibel sind, eingesetzt, da bei ausschließlicher Verwendung der Pyrazolonfarbstoffe der Formel (I) sowohl ein besonders gleichmäßiges Färbeergebnis als auch ein sehr gleichmäßiges Entfärbeergebnis erhalten wird.

Bei ausschließlicher Verwendung der Pyrazolonfarbstoffe der Formel (I) ist es nämlich möglich, die Haarfärbung zu jedem beliebigen Zeitpunkt wieder rückgängig zu machen. Von besonderem Vorteil ist hierbei, daß bei der Entfärbung die ursprüngliche Pigmentierung des behandelten Haares wiederhergestellt wird, wobei es in der Praxis unerheblich ist, ob die ursprüngliche Pigmentierung die natürliche Haarfarbe ist oder aber durch eine oxidative Haarfärbung erzielt wurde. Hierdurch ist es möglich sowohl die natürliche Haarfarbe als auch eine permanente Haaarfärbung für einen selbst gewählten Zeitraum zu ändern und nach Ablauf dieses Zeitraums in praktisch unveränderter Form wieder zurück zu erhalten.

Gegenstand der vorliegenden Anmeldung ist daher auch ein Verfahren zum temporären Färben von Haaren ("ON/OFF-Tönung"), bei dem das ungefärbte oder aber ein oxidativ gefärbtes Haar mit dem erfindungsgemäßen Färbemittel in der vorstehend beschriebenen Weise gefärbt wird und später ( zu einem beliebigen vom Anwender gewünschten Zeitpunkt) mit einem Reduktionsmittel oder Oxidationsmittel wieder entfärbt wird.

Die mit Pyrazolonfarbstoffen der Formel (I) erhaltenen Färbungen können beispielsweise reduktiv durch Einwirkung von geeigneten Reduktionsmitteln, wie zum Beispiel Sulfiten, Pyrosulfiten oder Hydrogensulfiten, beispielsweise Alkalisulfiten, Alkalihydrogensulfiten oder Alkalipyrosulfiten (wie zum Beispiel Natriumsulfit, Natriumpyrosulfit,

Kaliumpyrosulfit), Ammoniumsulfit oder Ammoniumhydrogensulfit, oder geeigneten Oxidationsmitteln, wie zum Beispiel handelsüblichen, Persulfate enthaltenden Blondierpulvern, vollständig entfärbt werden. Die Blondierpulver weisen in der Regel einen Gehalt von 5 bis 50 Gewichtsprozent, vorzugsweise 15 bis 30 Gewichtsprozent, Ammoniumpersulfat oder Alkalipersulfate oder einer Mischung aus Ammoniumpersulfat und Alkalipersulfaten auf. Die Entfärbung erfolgt vorzugsweise reduktiv unter Verwendung der vorgenannten Entfärbemittel (insbesondere mit Ammoniumsulfit oder Ammoniumhydrogensulfit),
wobei die Verwendung der vorgenannten Reduktionsmittel in Kombination mit anderen Reduktionsmitteln, wie zum Beispiel Reduktonen und/oder Thiolen, besonders bevorzugt ist.

Die Einwirkungszeit des Entfärbemittels beträgt hierbei je nach zu entfärbender Färbung und Temperatur (etwa 20 bis 50 Grad Celsius) 5 bis 45 Minuten, insbesondere 5 bis 30 Minuten, wobei durch Wärmezufuhr der Entfärbeprozeß beschleunigt werden kann. Nach Beendigung der Einwirkungszeit des Entfärbemittels wird das Haar mit Wasser gespült, gegebenenfalls mit einem Shampoo gewaschen und/oder einer Spülung, vorzugsweise einer neutralen oder schwach sauren Spülung, behandelt und sodann getrocknet, wobei sowohl das Shampoo als auch die Spülung ein Redukton, wie zum Beispiel Ascorbinsäure, enthalten kann.

Man erhält so nach der Entfärbung den ursprünglichen Farbton zurück. Verwendet man hingegen das Blondiermittel mit einer Wasserstoffperoxidlösung, vorzugsweise einer 6- bis 12prozentigen Wasserstoffperoxidlösung, so werden zum einen die Pyrazolonfarbstoffe der Formel (I) vollständig entfärbt, zum anderen aber wird das Haar zusätzlich aufgehellt ("blondiert").

Die nachstehenden Beispiele sollen den Erfindungsgegenstand näher erläutern, ohne ihn auf die angeführten Beispiele zu beschränken.

**Beispiele**

**Beispiel 1:** Verfahren zur Herstellung von 4-(4-(Bis-(2-hydroxyethyl)-amino)-benzyliden)-2-(2-hydroxyethyl)-5-methyl-2,4-dihydropyrazol-3-on (1)

2,85 g 5-Hydroxy-1-hydroxyethyl-3-methyl-1 H-pyrazol und 4-(Di-(2-hydroxyethyl)amino)-benzaldehyd werden 4 Stunden lang in Ethanol unter Rückfluß erhitzt. Anschließend wird das Reaktionsgemisch eingedampft und der Rückstand durch Zugabe geringer Mengen Isopropanol zur Kristallisation gebracht.

| | |
|---|---|
| Ausbeute: | 3,3 g 4-(4-(Bis-(2-hydroxyethyl)amino)-benzyliden)-2-(2-hydroxyethyl)-5-methyl-2,4-dihydro-pyrazol-3-on **(1)** |
| Schmelzpunkt: | 183-186 °C. |
| $\lambda_{max}$ ($H_2O$): | 466 nm |
| ε: | 39500 |
| $^1$H-NMR (DMSO): | $\delta$ = 8,55 (d, $^3J_{HH}$ = 9 Hz, 2H); 7,35 (s, 1 H); 6,85 (d, $^3J_{HH}$ = 9 Hz, 2H), 4,80 (m, 3H); 3,60 (m, 12H +Wasser); 2,10 ppm (s, 3H). |

| CHN-Analyse: | | | |
|---|---|---|---|
| ($C_{17}H_{23}N_3O_4$) | C | H | N |
| ber. | 61,25 | 6,95 | 12,60 |
| gef. | 61,23 | 7,01 | 12,51 |

**Beispiel 2:** Verfahren zur Herstellung von 2-(2-Hydroxyethyl)-5-methyl-4-thiophen-2-ylmethylen-2,4-dihydro-pyrazol-3-on (2)

2,85 g 5-Hydroxy-1-hydroxyethyl-3-methyl-1H-pyrazol und 2,25 g Thiophen-2-carbaldehyd werden in 30 ml 2-Butanol 7 Stunden lang unter Rückfluß erhitzt. Beim Abkühlen kristallisiert der leuchtend orange Farbstoff aus.

| | |
|---|---|
| Ausbeute: | 2,0 g 2-(2-Hydroxyethyl)-5-methyl-4-thiophen-2-ylmethylen-2,4-dihydro-pyrazol-3-on **(2)** |
| Schmelzpunkt: | 142-144 °C. |
| $\lambda_{max}$ (Methanol): | 349 nm, ε = 21600 |

| CHNS-Analyse: | | C | H | N | S |
|---|---|---|---|---|---|
| ($C_{11}H_{12}N_2O_2S$) | ber. | 55,91 | 5,12 | 11,86 | 13,57 |
| | gef. | 56,19 | 5,08 | 12,00 | 13,78 |

**Beispiel 3:** Verfahren zur Herstellung von 4-(4-Hydroxy-benzyliden)-2-(2-hydroxyethyl)-5-methyl-2,4-dihydro-pyrazol-3-on **(4)**

2,85 g 5-Hydroxy-1-hydroxyethyl-3-methyl-1H-pyrazol und 2,45 g 4-Hydroxy-benzaldehyd werden in 30 ml 2-Butanol unter Rückfluß erhitzt. Nach 16 Stunden wird abgekühlt, das Reaktionsgemisch auf 100 ml Essigester gegossen und filtriert. Man erhält 3,3 g eines Nebenproduktes, bei dem es sich vermutlich um das Acetal der nachfolgenden Formel handelt.

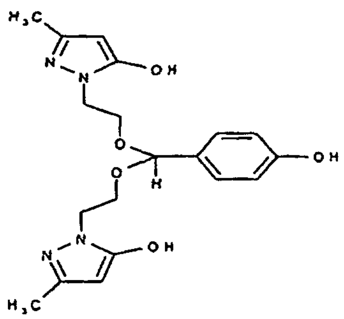

(Schmelzpunkt: 197-200°C; $^1$H-NMR (DMSO): δ = 6,95 (d, $^3J_{HH}$ = 7,5 Hz, 2H), 6,65 (d, $^3J_{HH}$ = 7,5 Hz, 2H), 4,6 (s,.1 H), 3,75 (m, 4H), 4,60 (m, 4H), 2.1 ppm (s, 6H)).

Die Mutterlauge wird eingedampft und der Rückstand durch Zugabe von Isopropanol zur Kristallisation gebracht.

| | |
|---|---|
| Ausbeute: | 1,6 g 4-(4-Hydroxy-benzyliden)-2-(2-hydroxyethyl)-5-methyl-2,4-dihydro-pyrazol-3-on (4) (leuchtend orange Kristalle) |
| Schmelzpunkt: | 207-210 °C |
| $^1$H-NMR (DMSO): | δ = 8,6 (d, $^3J_{HH}$ = 9 Hz, 2H); 7,4 (s, 1 H); 6,9 (d, $^3J_{HH}$ = 9 Hz, 2H); 3,65 (m, 4H); 2,15 ppm (s, 3H). |

**Beispiel 4:** Verfahren zur Herstellung von 4-(3-(4-Dimethylaminophenyl)-allyliden)-2-(2-hydroxyethyl)-5-methyl-2,4-dihydropyrazol-3-on (5)

2,85 g 5-Hydroxy-1-hydroxyethyl-3-methyl-1H-pyrazol und 3,5 g 4-Dimethylamino-zimtaldehyd werden in 30 ml 2-Butanol 14 Stunden lang unter Rückfluß erhitzt. Beim Abkühlen kristallisiert der Farbstoff aus.

| | |
|---|---|
| Ausbeute: | 1,9 g 4-(3-(4-Dimethylamino-phenyl)-allyliden)-2-(2-hydroxyethyl)-5-methyl-2,4-dihydro-pyrazol-3-on **(5)** |
| Schmelzpunkt: | 122 °C |
| $\lambda_{max}$ (Methanol): | 510 nm |
| ε: | 17600 |

**Beispiel 5:** Verfahren zur Herstellung von Triethylammonium-4-(4-(1 H-indol-3-ylmethylen)-3-methyl-5-oxo-4,5-dihydro-pyrazol-1-yl)-benzolsulfonat (6a)

2,55 g 5-Hydroxy-3-methyl-1-(4-sulfophenyl)-1H-pyrazol, 1,45 g Indol-3-carbaldehyd und 1,05 g Triethylamin werden in 25 ml Ethanol auf 60°C erhitzt. Nach 12 Stunden wird abgekühlt, nach Maßgabe der Kristallisation Aceton zugetropft und anschließend filtriert.

| | |
|---|---|
| Ausbeute: | 2,1 g Triethylammonium-4-(4-(1H-indol-3-ylmethylen)-3-methyl-5-oxo-4,5-dihydro-pyrazol-1-yl)-benzolsulfonat (6a) (rotbraunes Produkt) |

Schmelzpunkt: >250 °C.
$\lambda_{max}$ ($H_2O$): 406 nm
ε: 29500

| CHNS-Analyse: | | | | |
|---|---|---|---|---|
| ($C_{19}H_{15}N_3O_4S$ x $C_6H_{15}N$ x 0,5 $H_2O$) | | | | |
| ber. | 61,08 | 6,36 | 11,40 | 6,52 |
| gef. | 61,43 | 6,29 | 11,41 | 6,67 |

**Beispiel 6:** Verfahren zur Herstellung von Ammonium-4-(4-dimethylamino-benzyliden)-5-oxo-1-(4-sulfo-phenyl)-4,5-dihydro-1Hpyrazol-3-carbonsäure **(7a)**

2.85 g 5-Hydroxy-1-(4-sulfophenyl)-1H-pyrazol-3-carbonsäure, 1.65 g 4-Dimethylamino-benzaldehyd und 0.8 g Ammoniumacetat werden in 25 ml Ethanol auf 60°C erhitzt. Nach 7 Stunden wird abgekühlt und filtriert.

Ausbeute: 3.9 g Ammonium-4-(4-dimethylamino-benzyliden)-5-oxo-1-(4-sulfophenyl)-4,5-dihydro-1H-pyrazol-3-carbonsäure (7a) (orange-rotes Produkt)
Schmelzpunkt: >250 °C
$\lambda_{max}$ (Methanol): 482 nm
ε: 39000
$^1$H-NMR ($D_2O$): δ = 7,9 - 7,5 (m, 8H); 6,0 (d, 1 H); 2,30 ppm (s, 6H)

**Beispiel 7:** Verfahren zur Herstellung von Ammonium-4-(4-(2,4-dimethoxy-benzyliden)-3-methyl-5-oxo-4,5-dihydro-pyrazol-1-yl)-benzolsulfonat **(12a)**

2,55 g 5-Hydroxy-3-methyl-1-(4-sulfophenyl)-1H-pyrazol, 1,83 g 2,4-Dimethoxy-benzaldehyd und 0,8 g Ammoniumacetat werden in 25 ml Ethanol auf 70 °C erhitzt. Nach 5 Stunden wird abgekühlt und filtriert.

Ausbeute: 4,2 g Ammonium-4-(4-(2,4-dimethoxy-benzyliden)-3-methyl-5-oxo-4,5-dihydro-pyrazol-1-yl)-benzolsulfonat (12a) (orange-rotes Produkt)
Schmelzpunkt: >250°C.
$^1$H-NMR (DMSO): δ = 9,6 (d, $^3J_{HH}$ = 10 Hz, 1 H); 7,9 (d, $^3J_{HH}$ = 9 Hz, 2H); 7,85 (s, 1 H); 7,7 (d, $^3J_{HH}$ = 9 Hz, 2H); 7,2 (s, 4H); 6,65 (m, 2H); 3,9 (s, 3H); 3,85 (s, 3H); 2,25 ppm (s, 3H)

**Beispiel 8:** Verfahren zur Herstellung von Ammonium-4-(3-methyl-5-oxo-4-thiophen-2-ylmethylen-4,5-dihydro-pyrazol-1-yl)-benzolsulfonsäure **(13a)**

12,75 g 5-Hydroxy-3-methyl-1-(4-sulfophenyl)-1H-pyrazol, 6,2 g Thiophen-2-carbaldehyd und 3,85 g Ammoniumacetat werden in 100 ml Ethanol auf 80 °C erhitzt. Nach 4 Stunden wird abgekühlt und filtriert. Das Rohprodukt wird 30 min in 250 ml Methanol unter Rückfluß erhitzt und dann abgekühlt und filtriert.

Ausbeute: 15,6 g Ammonium-4-(3-methyl-5-oxo-4-thiophen-2-ylmethylen-4,5-dihydro-pyrazol-1-yl)-benzolsulfonsäure **(13a)** (oranges Produkt)
Schmelzpunkt: >250°C.
$\lambda_{max}$ (Methanol): 360 nm
ε: 66800
$^1$H-NMR (DMSO): δ = 8,35 (s, 1 H); 8,25 (d, $^3J_{HH}$ = 8,25 Hz, 2H); 8,05 (d, $^3J_{HH}$ = 8 Hz, 2H); 7,80 (d, $^3J_{HH}$ = 8 Hz, 2H); 7,40 (m, 5H); 2,35 ppm (s,3H)

**Beispiel 9:** Verfahren zur Herstellung von Natrium-2-(3-methyl-5-oxo-1-phenyl-1,5-dihydro-pyrazol-4-ylidenemethyl)-benzolsulfonat **(25a)**

2,1 g Benzaldehyd-2-sulfonsäure-natriumsalz, 1,75 g 3-Methyl-1-phenyl-2-pyrazolin-5-on und 1 g Triethylamin werden in 20 ml Etanol 5 Stunden lang unter Rückfluß erhitzt. Nach dem Abkühlen auf Raumtemperatur wird abgekühlt, der Niederschlag abfiltriert und getrocknet.

| | |
|---|---|
| Ausbeute: | 0,5 g Natrium-2-(3-methyl-5-oxo-1-phenyl-1,5-dihydro-pyrazol-4-ylidenemethyl)-benzolsulfonat **(25a)** (rotbraunes Pulver) |
| Schmelzpunkt: | > 250°C |
| λmax (H2O): | 361 nm |
| ε: | 17600 |
| $^1$H-NMR (90 MHz, D2O): | d = 8,7 - 7.0 (m, 10H); 2,3 ppm (s, 3H). |

**Beispiel 10:** Färbemittel

| | |
|---|---|
| 10,0 g | Ethanol |
| 10,0 g | Polyoxyethylenlaurylether (25%ige wäßrige Lösung) |
| 0,8 g | 4-(4-(Bis-(2-hydroxyethyl)amino)-benzyliden)-2-(2- hydroxyethyl)-5-methyl-2,4-dihydro-pyrazol-3-on (1) |
| ad 100,0 g | Wasser, demineralisiert |

Die Färbelösung wird auf gebleichtes Haar aufgetragen. Nach 20 Minuten Einwirkungszeit bei 40 °C wird mit Wasser ausgewaschen und getrocknet. Das Haar erhält einen leuchtend orangen Farbton.

Zur Entfärbung wird das Haar anschließned mit einer wäßrigen Lösung des in der nachfolgenden Tabelle genannten Reduktionsmittels behandelt. Nach wenigen Minuten tritt die Entfärbung ein. Das Haar wird mit Wasser gründlich ausgespült und anschließend getrocknet.
Das entfärbte Haar ist annähernd weiß.

| | L* | a* | b* | $\Delta E_{1/2}$ | Entfärbungsgrad [%] |
|---|---|---|---|---|---|
| unbehandeltes Haar | 84,80 | -0,75 | 11,53 | | |
| gefärbtes Haar | 60,13 | 48,54 | 70,96 | 81,06 | |
| mit 10% $Na_2SO_3$ entfärbtes Haar | 80,36 | 0,07 | 15,41 | 76,45 | 94 |

Beispiel 11: Färbemittel

| | |
|---|---|
| 10,0 g | Ethanol |
| 10,0 g | Polyoxyethylenlaurylether (25%ige wäßrige Lösung) |
| 0,6 g | 4-(4-Hydroxy-benzyliden)-2-(2-hydroxyethyl)-5-methyl-2,4-dihydro-pyrazol-3-on (4) |
| ad 100,0 g | Wasser, demineralisiert |

Die Färbelösung wird auf gebleichtes Haar aufgetragen. Nach 20 Minuten Einwirkzeit bei 40 °C wird mit Wasser ausgewaschen und getrocknet. Das Haar erhält einen zitronengelben Farbton.

Zur Entfärbung wird das Haar jeweils mit einer wäßrigen Lösung des in der nachfolgenden Tabelle genannten Reduktionsmittels behandelt. Nach wenigen Minuten tritt die Entfärbung ein. Das Haar wird mit Wasser gründlich ausgespült und anschließend getrocknet. Das entfärbte Haar ist weiß.

| | L* | a* | b* | $\Delta E_{1/2}$ | Entfärbungsgrad [%] |
|---|---|---|---|---|---|
| unbehandeltes Haar | 84,80 | -0,75 | 11,53 | | |
| gefärbtes Haar | 77,86 | 6,81 | 80,21 | 69,44 | |
| mit 10% $Na_2SO_3$ entfärbtes Haar | 82,98 | 0,69 | 11,54 | 69,13 | 100 |

**Beispiel 12:** Färbemittel

| | |
|---|---|
| 5,0 g | Ethanol |
| 1,5 g | Glycolsäure |
| 2,0 g | Natrium-cocoamphoacetat (50%ige wäßrige Lösung) |
| 5,0 g | Benzylalkohol |
| 1,1 g | Ammonium-4-(4-dimethylamino-benzyliden)-5-oxo-1-(4-sulfophenyl)-4,5-dihydro-1 H-pyrazol-3-carbonsäure **(7a)** |
| ad 100,0 g | Wasser, demineralisiert |

Die Färbelösung wird auf gebleichtes Haar aufgetragen. Nach 20 Minuten Einwirkzeit bei 40 °C wird mit Wasser ausgewaschen und getrocknet. Das Haar erhält einen leuchtend orange-roten Farbton.

Zur Entfärbung wird das Haar jeweils mit einer wäßrigen Lösung des in der nachfolgenden Tabelle genannten Reduktionsmittels behandelt. Nach wenigen Minuten tritt die Entfärbung ein. Das Haar wird mit Wasser gründlich ausgespült und anschließend getrocknet.
Nach der Entfärbung bleibt ein hellbeiger Schimmer.

| | L* | a* | b* | $\Delta E_{1/2}$ | Entfärbungsgrad [%] |
|---|---|---|---|---|---|
| unbehandeltes Haar | 84,80 | -0,75 | 11,53 | | |
| gefärbtes Haar | 45,58 | 64,70 | 45,45 | 83,50 | |
| mit 10% $Na_2SO_3$ entfärbtes Haar | 83,36 | 7,56 | 17,28 | 74,07 | 89 |

**Beispiel 13:** Färbemittel

Ein gemäß dem zuvor angeführten Beispiel gefärbtes Haar wird 8 Minuten bei 20°C mit einer Lösung, die 5% Natriumsulfit und 5% Ascorbinsäure enthält, behandelt. Das Haar ist deutlich heller als das nach Beispiel 11 entfärbte Haar.

| | L* | a* | b* | $\Delta E_{1/2}$ | Entfärbungsgrad [%] |
|---|---|---|---|---|---|
| unbehandeltes Haar | 84,80 | -0,75 | 11,53 | | |
| gefärbtes Haar | 45,58 | 64,70 | 45,45 | 83,50 | |
| mit 5% $Na_2SO_3$/ 5% Ascorbinsäure entfärbtes Haar | 85,07 | 3,08 | 16,98 | 78,53 | 94 |

**Beispiel 14**: Färbemittel

| | |
|---|---|
| 5,0 g | Ethanol |
| 1,5 g | Glycolsäure |
| 2,0 g | Natrium-cocoamphoacetat (50%ige wäßrige Lösung) |
| 5,0 g | Benzylalkohol |
| 1,0 g | Ammonium-4-(4-(2,4-dimethoxy-benzyliden)-3-methyl-5-oxo-4,5-dihydro-pyrazol-1-yl)-benzolsulfonat **(12a)** |
| ad 100,0 g | Wasser, demineralisiert |

Die Färbelösung wird auf gebleichtes Haar aufgetragen. Nach 20 Minuten Einwirkzeit bei 40 °C wird mit Wasser ausgewaschen und getrocknet. Das Haar erhält einen leuchtend gelb-orangen Farbton.

Zur Entfärbung wird das Haar jeweils mit einer wäßrigen Lösung des in der nachfolgenden Tabelle genannten Reduktionsmittels behandelt. Nach wenigen Minuten tritt die Entfärbung ein. Das Haar wird mit Wasser gründlich ausgespült und anschließend getrocknet.
Das entfärbte Haar ist weiß.

| | L* | a* | b* | $\Delta E_{1/2}$ | **Entfärbungsgrad [%]** |
|---|---|---|---|---|---|
| unbehandeltes Haar | 84,80 | -0,75 | 11,53 | | |
| gefärbtes Haar | 70,34 | 27,18 | 82,45 | 77,58 | |
| mit 10% $Na_2SO_3$ entfärbtes Haar | 86,80 | -1,31 | 13,95 | 75,99 | 98 |

**Beispiel 15:** Färbemittel

| | |
|---|---|
| 5,0 g | Ethanol |
| 1,5 g | Glycolsäure |
| 2,0 g | Natrium-cocoamphoacetat (50%ige wäßrige Lösung) |
| 5,0 g | Benzylalkohol |
| 1,0 g | Ammonium-4-(3-methyl-5-oxo-4-thiophen-2-ylmethylen- 4,5-dihydro-pyrazol-1-yl)-benzolsulfonsäure **(13a)** |
| ad 100,0 g | Wasser, demineralisiert |

Die Färbelösung wird auf gebleichtes Haar aufgetragen. Nach 20 Minuten Einwirkzeit bei 40 °C wird mit Wasser ausgewaschen und getrocknet. Das Haar erhält einen goldgelben Farbton.

Zur Entfärbung wird das Haar jeweils mit einer wäßrigen Lösung des in der nachfolgenden Tabelle genannten Reduktionsmittels behandelt. Nach wenigen Minuten tritt die Entfärbung ein. Das Haar wird mit Wasser gründlich ausgespült und anschließend getrocknet. Das Haar ist weiß.

| | L* | a* | b* | $\Delta E_{1/2}$ | **Entfärbungsgrad [%]** |
|---|---|---|---|---|---|
| unbehandeltes Haar | 84,80 | -0,75 | 11,53 | | |
| gefärbtes Haar | 67,65 | 32,34 | 69,30 | 68,75 | |
| mit 10% $Na_2SO_3$ entfärbtes Haar | 85,80 | -1,15 | 15,79 | 65,68 | 96 |

**Beispiel 16:** Färbemittel

| | |
|---|---|
| 5,0 g | Ethanol |
| 1,5 g | Glycolsäure |
| 2,0 g | Natrium-cocoamphoacetat (50%ige wäßrige Lösung) |
| 5,0 g | Benzylalkohol |
| 0,8 g | Ammonium-4-(4-(3-(4-dimethylamino-phenyl)-allyliden)-3-methyl-5-oxo-4,5-dihydro-pyrazol-1-yl)-benzolsulfonat **(16a)** |
| ad 100,0 g | Wasser, demineralisiert |

Die Färbelösung wird auf gebleichtes Haar aufgetragen. Nach 20 Minuten Einwirkzeit bei 40 °C wird mit Wasser ausgewaschen und getrocknet. Das Haar erhält einen weinroten Farbton.

Zur Entfärbung wird das Haar jeweils mit einer wäßrigen Lösung des in der nachfolgenden Tabelle genannten Reduktionsmittels behandelt. Nach wenigen Minuten tritt die Entfärbung ein. Das Haar wird mit Wasser gründlich ausgespült und anschließend getrocknet.
Das entfärbte Haar behält einen rosa Schimmer.

| | L* | a* | b* | $\Delta E_{1/2}$ | Entfärbungsgrad [%] |
|---|---|---|---|---|---|
| unbehandeltes Haar | 84,80 | -0,75 | 11,53 | | |
| gefärbtes Haar | 18,64 | 16,61 | -0,55 | 69,46 | |
| mit 10% $Na_2SO_3$ entfärbtes Haar | 73,74 | 11,75 | 3,99 | 55,50 | 80 |

**Beispiel 17:** Färbemittel

| | |
|---|---|
| 5,0 g | Ethanol |
| 1,5 g | Glycolsäure |
| 2,0 g | Natrium-cocoamphoacetat (50%ige wäßrige Lösung) |
| 5,0 g | Benzylalkohol |
| 0,9 g | Natrium-2-(3-methyl-5-oxo-1-phenyl-1,5-dihydro-pyrazol-4-ylidenemethyl)-benzolsulfonat **(25a)** |
| ad 100,0 g | Wasser, demineralisiert |

Die Färbelösung wird auf gebleichtes Haar aufgetragen. Nach 20 Minuten Einwirkzeit bei 40 °C wird mit Wasser ausgewaschen und getrocknet. Das Haar erhält einen rehbraunen Farbton.

Zur Entfärbung wird das Haar jeweils mit einer wäßrigen Lösung des in der nachfolgenden Tabelle genannten Reduktionsmittels behandelt. Nach wenigen Minuten tritt die Entfärbung ein. Das Haar wird mit Wasser gründlich ausgespült und anschließend getrocknet.
Das entfärbte Haar ist hellbeige.

| | L* | a* | b* | $\Delta E_{1/2}$ | Entfärbungsgrad [%] |
|---|---|---|---|---|---|
| unbehandeltes Haar | 84,80 | -0,75 | 11,53 | | |
| gefärbtes Haar | 47,02 | 42,51 | 35,69 | 62,31 | |
| mit 10% $Na_2SO_3$ entfärbtes Haar | 81,95 | 5,73 | 19,23 | 53,33 | 86 |

**Beispiel 18:** Färbemittel

| | |
|---|---|
| 5,0 g | Ethanol |
| 1,5 g | Glycolsäure |
| 2,0 g | Natrium-cocoamphoacetat (50%ige wäßrige Lösung) |
| 5,0 g | Benzylalkohol |
| 1,1 g | Ammonium-4-chlor-3-(4-(4-dimethylamino-benzyliden)-3-methyl-5-oxo-4,5-dihydro-pyrazol-1-yl)-benzolsulfonat **(9a)** |
| ad 100,0 g | Wasser, demineralisiert |

Die Färbelösung wird auf mittelblondes Haar aufgetragen. Nach 20 Minuten Einwirkzeit bei 40 °C wird mit Wasser ausgewaschen und getrocknet. Das Haar erhält einen kupferbraunen Farbton.

Zur Entfärbung wird das Haar jeweils mit einer wäßrigen Lösung des in der nachfolgenden Tabelle genannten Entfärbungsmittels behandelt (*) Anmerkung: Der berechnete Entfärbegrad von 253 % setzt sich zusammen aus der Summe der Entfärbung des applizierten Farbstoffs sowie der zusätzlichen Aufhellung der natürlichen Haarfarbe ("Blondierung")). Nach wenigen Minuten tritt die Entfärbung ein. Das Haar wird mit Wasser gründlich ausgespült und anschließend getrocknet.

| | L* | a* | b* | $\Delta E_{1/2}$ | Entfärbungsgrad [%] |
|---|---|---|---|---|---|
| unbehandeltes Haar | 36,18 | 7,89 | 14,25 | | |
| gefärbtes Haar | 30,21 | 19,12 | 19,76 | 13,86 | |
| mit 5% $Na_2SO_3$ / 5% Ascorbinsäure behandeltes Haar | 35,33 | 8,90 | 15,86 | 12,08 | 87 |
| oxidativ behandeltes Haar (Mischung aus 45% Kaliumpersulfat + 6% Ammoniumpersulfat / Wasser 1:1) | 36,64 | 9,96 | 17,33 | 11,45 | 83 |
| blondiertes Haar (Mischung aus 45% Kaliumpersulfat + 6% Ammoniupersulfat/ 6% Wasserstoffperoxid 1:1) | 62,44 | 10,02 | 30,00 | 35,02 | 253*) |

**Beispiel 19:** Umfärbung auf oxidativ vorgefärbtem Haar

Gebleichtes Haar, das mit einem handelsüblichen Oxidationshaarfärbemittel mittelblond gefärbt worden ist, wird mit einem Färbemittel gemäß Beispiel 17 behandelt. Nach 20 Minuten Einwirkzeit bei 40 °C wird mit Wasser ausgewaschen und getrocknet. Das Haar ist in einem mittleren Kupferton gefärbt.

Zur Entfärbung wird das Haar gemäß der nachfolgenden Tabelle behandelt. Nach wenigen Minuten erhält man den Farbton des vorgefärbten Haares bei einer zusätzlichen Aufhellung zurück. Da des weiteren eine merkliche Farbabweichung der Oxidationshaarfarbe zu verzeichnen war, ist eine Berechnung des Entfärbegrades nicht sinnvoll.

| | L* | a* | b* | Farbton |
|---|---|---|---|---|
| oxidativ gefärbtes Haar | 37,24 | 4,76 | 12,78 | mittelblond |
| gemäß Beispiel 18 gefärbtes Haar | 28,77 | 23,94 | 17,42 | kupfer |
| oxidativ behandeltes Haar (Mischung aus 45%Kaliumpersulfat + 6% Ammonium - persulfat / Wasser 1:1) | 44,45 | 14,91 | 23,65 | ähnlich Farbton des oxidativ gefärbten Haares |
| blondiertes Haar (Mischung aus 45% Kaliumpersulfat + 6% Ammonium- persulfat / 6% Wasserstoffperoxid 1:1) | 77,69 | 5,64 | 20,72 | hellblond |
| mit 5% $Na_2SO_3$ / 5% Ascorbinsäure behandeltes Haar | 46.53 | 7,81 | 21,65 | ähnlich Referenzton |

**Beispiel 20 :** Färbemittel mit Carrier

| | |
|---|---|
| 5,0 g | Ethanol |
| 2,0 g | Milchsäure |
| 2,0 g | Natrium-cocoamphoacetat (50prozentige wäßrige Lösung) |
| 5,0 g | Carrier gemäß nachfolgender Tabelle |
| 1,0 g | Ammonium-4-(4-dimethylamino-benzyliden)-5-oxo-1-(4-sulfophenyl)-4,5-dihydro-1H-pyrazol-3-carbonsäure **(7a)** |
| ad 100,0 g | Wasser, demineralisiert |

Die Färbelösung wird auf gebleichtes Haar aufgetragen. Nach 20 Minuten Einwirkzeit bei 40 °C wird mit Wasser ausgewaschen und getrocknet. Das Mittel ohne Carrier ergibt einen hellen Orangeton während die Mittel mit Carrier zu intensiven Orangerot-Tönen führen.

| Beispiel | Carrier | L* | a* | b* |
|---|---|---|---|---|
| a | - | 61,94 | 42,19 | 44,18 |

(fortgesetzt)

| Beispiel | Carrier | L* | a* | b* |
|---|---|---|---|---|
| b | Isovanillin | 47,70 | 65,56 | 48,05 |
| c | 2-Hydroxymethyl-thiophen | 46,51 | 65,71 | 47,71 |
| d | Benzylalkohol | 45,58 | 64,70 | 45,45 |

Die in den vorliegenden Beispielen angegebenen L*a*b*-Farbmesswerte wurden mit einem Farbmessgerät der Firma Minolta, Typ Chromameter II, ermittelt.

Hierbei steht der L-Wert für die Helligkeit (das heißt je geringer der L-Wert ist, umso größer ist die Farbintensität), während der a-Wert ein Maß für den Rotanteil ist (das heißt je größer der a-Wert ist, umso größer ist der Rotanteil). Der b-Wert ist ein Maß für den Blauanteil der Farbe, wobei der Blauanteil umso größer ist, je negativer der b-Wert ist.

Der Wert ΔE gibt die Farbdifferenz an, die zwischen dem unbehandelten und dem gefärbten Haar beziehungsweise dem gefärbten und dem entfärbten Haar besteht. Er wird folgendermaßen bestimmt:

$$\Delta E = \sqrt{(L_i-L_0)^2 + (a_i-a_0)^2 + (b_i-b_o)^2}$$

wobei $L_0$, $a_0$ und $b_0$ die Farbmesswerte vor der Färbung beziehungsweise Entfärbung bedeuten und $L_i$, $a_i$ und $b_i$ die Werte nach der Färbung beziehungsweise Entfärbung bedeuten.

Der Entfärbegrad in Prozent wurde nach der folgenden Formel ermittelt:

$$\text{Entfärbegrad [\%]} = (\Delta E_2/\ \Delta E_1) \times 100$$

Hierbei steht $\Delta E_1$ für den Färbeschritt und $\Delta E_2$ für den Entfärbeschritt.
Die Berechnung kann dabei nur als Bestätigung des visuellen Eindrucks dienen, da ΔE ausschließlich den Betrag der Farbdifferenz angibt, jedoch nicht deren Änderungsrichtung.

Alle in der vorliegenden Anmeldung genannten Prozentangaben stellen soweit nicht anders angegeben Gewichtsprozente dar.

**Patentansprüche**

1. Mittel zur Färbung von Keratinfasem, **dadurch gekennzeichnet, daß** es mindestens einen Pyrazolonfarbstoff der allgemeinen Formel (I) oder dessen physiologisch verträgliches Salz enthält,

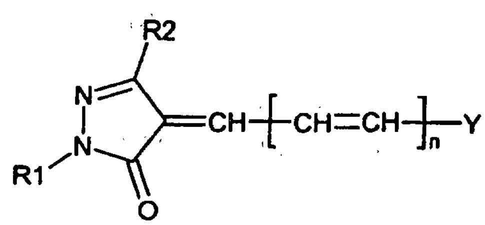

(I)

worin **R1** Wasserstoff, eine geradkettige oder verzweigte C1- bis C8-Alkylgruppe, eine Hydroxyethylgruppe, eine Dihydroxypropylgruppe, eine Methoxyethylgruppe, eine Carboxyethylgruppe, eine C1- bis C4-Sulfoalkylgruppe, einen Phenylrest, einen mit einem oder mehreren Halogenatomen substituierten Phenylrest, einen mit einer oder zwei Sulfonsäuregruppen substituierten Phenylrest, einen mit einer oder zwei Carbonsäuregruppen substituierten Phenylrest, einen mit einer oder mehreren unverzweigten oder verzweigten C1- bis C8-Alkylgruppen substituierten Phenylrest, einen mit einer oder mehreren unverzweigten oder verzweigten C1- bis C8-Alkoxygruppen substituierten Phenylrest, einen Benzylrest, einen mit einem oder mehreren Halogenatomen substituierten Benzylrest, einen mit einer C1- bis C4-Alkylgruppe substituierten Benzylrest, einen mit einer Hydroxygruppe substituierten Benzylrest, einen mit einer Methoxygruppe substituierten Benzylrest, einen mit einer Carboxygruppe substituierten Benzylrest, einen mit einer Nitrogruppe substituierten Benzylrest, einen mit einer Aminogruppe substituierten Benzylrest oder einen fünfgliedrigen oder sechsgliedrigen gesättigten oder ungesättigten Heterocyclus bedeutet;
R2 Wasserstoff, eine verzweigte oder unverzweigte C1- bis C6-Alkylgruppe, einen Phenylrest, eine Aminogruppe, eine acylierte oder sulfonylierte Aminogruppe, eine Acetylgruppe, eine Methoxygruppe, eine Carbonsäuregruppe, eine mit einem geradkettigen oder verzweigten C1-bis C8-Alkohol oder einem Ethylenglykolmonomethylether oder einem Ethylenglykolmonoethylether veresterte Carbonsäuregruppe, eine Carbonsäureamidgruppe, eine Carbonsäureanilidgruppe oder eine 2-Amino-2-oxyethylgruppe bedeutet;
Y einen aromatischen fünfgliedrigen oder sechsgliedrigen carbocyclischen oder heterocyclischen Ring oder einen benzoanellierten aromatischen fünfgliedrigen oder sechsgliedrigen carbocyclischen oder heterocyclischen Ring darstellt, oder Y für einen Rest der allgemeinen Formel (II) oder (III),

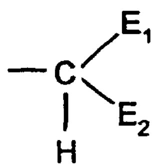
(II),

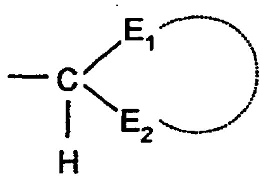
(III),

in der **E1** und **E2** unabhängig voneinander eine Nitrilgruppe, eine Alkylsulfonylgruppe, eine Acylgruppe, eine Carbonestergruppe oder eine Carbonamidgruppe bedeuten, steht; und n gleich 0, 1 oder 2 ist.

**2.** Mittel nach Anspruch 1 , **dadurch gekennzeichnet, daß** der Pyrazolonfarbstoff der Formel (I) ausgewählt ist aus Verbindungen der Formel (I) mit
**R1** gleich einer geradkettigen oder verzweigten C1- bis C4- Alkylgruppe, einer Hydroxyethylgruppe, einer Dihydroxypropylgruppe, einer Methoxyethylgruppe, einer C1- bis C4-Sulfoalkylgruppe, einem Phenylrest, einem mit einem oder mehreren Halogenatomen substituierten Phenylrest, einem mit einer oder zwei Sulfonsäuregruppen substituierten Phenylrest oder einem mit einer oder zwei Carbonsäuregruppen substituierten Phenylrest;
R2 gleich Wasserstoff, einer Methylgruppe, einer Carbonsäuregruppe, einer mit einem geradkettigen C1- bis C4-Alkohol veresterten Carbonsäuregruppe oder einer Carbonsäureamidgruppe;
**Y** gleich einem Donor-substituierten Phenylrest, einem aromatischen fünfgliedrigen heterocyclischen Ring oder einem benzoanellierten aromatischen fünfgliedrigen heterocyclischen Ring; und
n gleich 0, 1 oder 2.

**3.** Mittel nach Anspruch 1 oder 2, **dadurch gekennzeichnet, daß** das physiologisch verträgliche Salz ausgewählt ist aus Pyridiniumsalzen, Tetraalkylphosphoniumsalzen, Tetraarylphosphoniumsalze, Ammoniumsalzen, Triethylammoniumsalzen, Natriumsalzen, Kaliumsalzen, N-Methylmorpholiniumsalzen, Monoethanolammoniumsalzen, Diethanolammoniumsalzen und Triethanolammoniumsalzen.

**4.** Mittel nach einem der Ansprüche 1 bis 3, **dadurch gekennzeichnet, daß** der Polymethinfarbstoff der Formel (I) ausgewählt ist aus 4-(4-(Bis-(2-hydroxyethyl)amino)-benzyliden)-2-(2-hydroxyethyl)-5-methyl-2,4-dihydro-pyrazol-3-on; 2-(2-Hydroxyethyl)-5-methyl-4-thiophen-2-ylmethylen-2,4-dihydro-pyrazol-3-on; 2-(2-hydroxyethyl)-5-methyl-4-thiophen-3-ylmethylen-2,4-dihydro-pyrazol-3-on; 4-(4-Hydroxybenzyliden)-2-(2-hydroxyethyl)-5-methyl-2,4-dihydro-pyrazol-3-on; 4-(3-(4-Dimethylamino-phenyl)-allyliden)-2-(2-hydroxyethyl)-5-methyl-2,4-dihydropyrazol-3-on; 4-(4-(1H-Indol-3-ylmethylen)-3-methyl-5-oxo-4,5-dihydro-pyrazol-1-yl)-benzolsulfonsäure; Triethylammonium-4-(4-(1Hindol-3-ylmethylen)-3-methyl-5-oxo-4,5-dihydro-pyrazol-1-yl)-benzolsulfonat; 4-(4-Dimethylamino-benzyliden)-5-oxo-1-(4-sulfo-phenyl)-4,5-dihydro-1H-pyrazol-3-carbonsäure; Ammonium-4-(4-dimethylaminobenzyliden)-5-oxo-1-(4-sulfo-phenyl)-4,5-dihydro-1H-pyrazol-3-carbonsäure; 4-(4-(4-Dimethylamino-benzyliden)-3-methyl-5-oxo-4,5-dihydro-pyrazol-1-yl)-benzolsulfonsäure; Ammonium-4-(4-(4-dimethylamino-benzyliden)-3-methyl-5-oxo-4,5-dihydro-pyrazol-1-yl)-benzolsulfonat; 4-Chlor-3-(4-(4-dimethylamino-benzyliden)-3-methyl-5-oxo-4,5-dihydro-pyrazol-1-yl)-benzolsulfonsäure; Ammonium-4-chlor-3-(4-(4-dimethylamino-benzyliden)-3-methyl-5-oxo-4,5-dihydro-pyrazol-1-yl)-benzolsulfonat; 4-(4-(4-Hydroxy-3-methoxy-benzyliden)-3-methyl-5-oxo-4,5-dihydro-pyrazol-1-yl)-benzolsulfonsäure; 4-(4-(4-Methoxy-benzyliden)-3-methyl-5-oxo-4,5-dihydro-pyrazol-1-yl)-benzolsulfonsäure;4-(4-(2,4-Dimethoxy-benzyliden)-3-methyl-5-oxo-4,5-dihydro-pyrazol-1-yl)-benzolsulfonsäure; Ammonium-4-(4-(2,4-dimethoxy-benzyliden)-3-methyl-5-oxo-4,5-dihydro-pyrazol-1-yl)-benzolsulfonat; 4-(3-Methyl-5-oxo-4-thiophen-2-ylmethylen-4,5-dihydro-pyrazol-1-yl)-benzolsulfonsäure; Ammonium-4-(3-methyl-5-oxo-4-thiophen-2-yl-methylen-4,5-dihydropyrazol-1-yl)-benzolsulfonsäure; 4-(3-Methyl-4-(1-methyl-1,2,3,4-tetrahydro-chinolin-6-ylmethylen)-5-oxo-4,5-dihydro-pyrazol-1-yl)-benzolsulfonsäure; 4-(4-(1-(2-Cyan-ethyl)-1,2,3,4-tetrahydro-chinolin-6-ylmethylen)-3-methyl-5-oxo-4,5-dihydro-pyrazol-1-yl)-benzolsulfonsäure;

4-(4-(3-(4-Dimethylamino-phenyl)-allyliden)-3-methyl-5-oxo-4,5-dihydropyrazol-1-yl)-benzolsulfonsäure; Ammonium-4-(4-(3-(4-dimethylaminophenyl)-allyliden)-3-methyl-5-oxo-4,5-dihydro-pyrazol-1-yl)-benzolsulfonat; 4-(3-(4-Dimethylamino-phenyl)-allyliden)-5-oxo-1-(4-sulfophenyl)-4,5-dihydro-1H-pyrazol-3-carbonsäure; Ammonium-4-(3-(4-dimethylaminophenyl)-allyliden)-5-oxo-1-(4-sulfophenyl)-4,5-dihydro-1H-pyrazol-3-carbonsäure; 3-(5-Hydroxy-3-methyl-4-(2,4,6-trioxo-tetrahydropyrimidin-5-ylidenmethyl)-pyrazol-1-yl)-benzolsulfonsäure; 5-(1-(2,5-Dichlorphenyl)-5-hydroxy-3-methyl-1 H-pyrazol-4-ylmethylen)-2-thioxo-dihydro-pydmidin-4,6-dion; 4-(4-(2,2-Dimethyl-4,6-dioxo-[1,3]dioxan-5-ylidenmethyl)-5-hydroxy-3-methyl-pyrazol-1-yl)-benzolsulfonsäure; 4-(4-(5-(4,6-Dioxo-1,3-dipropyl-2-thioxo-tetrahydro-pyrimidin-5-yliden)-penta-1,3-dienyl)-5-hydroxy-3-methyl-pyrazol-1-yl)-benzolsulfonsäure; 2-(4-(4,4-Bis-ethoxy-carbonylbuta-1,3-dienyl)-5-hydroxy-3-methyl-pyrazol-1-yl)-terephthalsäure; 1-(4-Carboxy-phenyl)-4-(4,4-dicarbamoyl-buta-1,3-dienyl)-5-hydroxy-1Hpyrazol-3-carbonsäure; 2-(4-(2,2-Bis-ethansulfonyl-vinyl)-5-hydroxy-3-methyl-pyrazol-1-yl)-terephthalsäure; 2-(3-Methyl-5-oxo-1-phenyl-1,5-dihydro-pyrazol-4-ylidenemethyl)-benzolsulfonsäure und Natrium-2-(3-methyl-5-oxo-1-phenyl-1,5-dihydropyrazol-4-ylidenemethyl)-benzolsulfonat.

**5.** Mittel nach einem der Ansprüche 1 bis 4, **dadurch gekennzeichnet, daß** es 0,01 bis 5 Gewichtsprozent des Polymethinfarbstoffes der Formel (I) enthält.

**6.** Mittel nach einem der Ansprüche 1 bis 5, **dadurch gekennzeichnet, daß** es einen pH-Wert von 2 bis 11 aufweist.

**7.** Mittel nach einem der Ansprüche 1 bis 6, **dadurch gekennzeichnet, daß** es einen Carrier enthält.

**8.** Mittel nach einem der Ansprüche 1 bis 7, **dadurch gekennzeichnet, daß** es ein Haarfärbemittel ist.

**9.** Mittel nach einem der Ansprüche 1 bis 8, **dadurch gekennzeichnet, daß** es zusätzlich natürliche oder synthetische Polymere oder modifizierte Polymere natürlichen Ursprungs enthält.

**10.** Verfahren zum temporären Färben von Haaren, **dadurch gekennzeichnet, daß** 50 bis 150 Gramm eines Mittels nach einem der Ansprüche 1 bis 8 auf das Haar aufgetragen werden und nach einer Einwirkungszeit von 10 bis 45 Minuten bei 20 bis 50 °C das Haar mit Wasser gespült wird, gegebenenfalls mit einem Shampoo gewaschen und/oder mit der wäßrigen Lösung einer schwachen organischen Säurewie zum Beispiel Zitronensäure oder Weinsäure nachgespült und sodann getrocknet wird, und das gefärbte Haar zu einem späteren Zeitpunkt mit Hilfe eines Reduktionsmittels oder Oxidationsmittels wieder entfärbt wird.

**11.** Verfahren zum temporären Färben von Haaren, **dadurch gekennzeichnet, daß** das Haar mit einem Mittel nach Anspruch 9 befeuchtet wird, zur Frisur gelegt und anschließend getrocknet wird, und das gefärbte Haar zu einem späteren Zeitpunkt mit Hilfe eines Reduktionsmittels oder Oxidationsmittels wieder entfärbt wird.

**12.** Verfahren nach Anspruch 10 oder 11, **dadurch gekennzeichnet, daß** als Reduktionsmittel Alkalisulfite, Alkalihydrogensulfite, Alkalihyposulfite, Alkalipyrosulfite, Ammoniumsulfit oder Ammoniumhydrogensulfit, verwendet werden.

**13.** Verfahren nach Anspruch 10 oder 11, **dadurch gekennzeichnet, daß** als Reduktionsmittel eine Kombination aus a) Alkalisulfiten, Alkalihydrogensulfiten, Alkalipyrosulfiten, Ammoniumsulfit oder Ammoniumhydrogensulfit und b) Reduktonen und/oder Thiolen, verwendet wird.

**14.** Verfahren nach Anspruch 10 oder 11, **dadurch gekennzeichnet, daß** als Oxidationsmittel 5 bis 50 Gewichtsprozent Ammoniumpersulfat und/oder Alkalipersulfate enthaltende Blondierpulver, alleine oder in Kombination mit einer Wasserstoffperoxidlösung, verwendet werden.

## Claims

**1.** Agent for colouring keratin fibres, **characterized in that** it comprises at least one pyrazolone dye of the general formula (I) or physiologically compatible salt thereof,

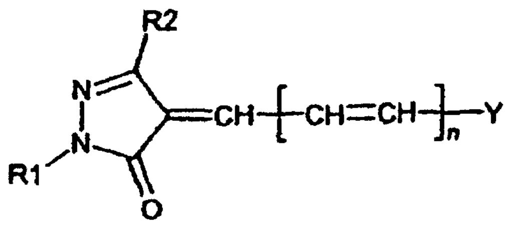

(I)

in which **R1** is hydrogen, a straight-chain or branched C1- to C8-alkyl group, a hydroxyethyl group, a dihydroxypropyl group, a methoxyethyl group, a carboxyethyl group, a C1- to C4-sulphoalkyl group, a phenyl radical, a phenyl radical substituted by one or more halogen atoms, a phenyl radical substituted by one or two sulphonic acid groups, a phenyl radical substituted by one or two carboxylic acid groups, a phenyl radical substituted by one or more unbranched or branched C1-to C8-alkyl groups, a phenyl radical substituted by one or more unbranched or branched C1- to C8-alkoxy groups, a benzyl radical, a benzyl radical substituted by one or more halogen atoms, a benzyl radical substituted by a C1- to C4-alkyl group, a benzyl radical substituted by a hydroxyl group, a benzyl radical substituted by a methoxy group, a benzyl radical substituted by a carboxyl group, a benzyl radical substituted by a nitro group, a benzyl radical substituted by an amino group, or a five-membered or six-membered saturated or unsaturated heterocycle;
**R2** is hydrogen, a branched or unbranched C1- to C6-alkyl group, a phenyl radical, an amino group, an acylated or sulphonylated amino group, an acetyl group, a methoxy group, a carboxylic acid group, a carboxylic acid group esterified with a straight-chain or branched C1- to C8-alcohol or an ethylene glycol monomethyl ether or an ethylene glycol monoethyl ether, a carboxamide group, a carboxanilide group or a 2-amino-2-oxyethyl group;
**Y** is an aromatic five-membered or six-membered carbocyclic or heterocyclic ring or a benzo-fused aromatic five-membered or six-membered carbocylic or heterocyclic ring, or **Y** is a radical of the general formula (II) or (III),

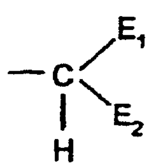

(II),

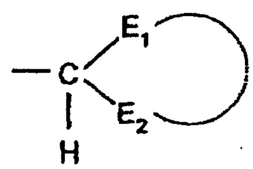

(III),

in which **E1** and **E2**, independently of one another, are a nitrile group, an alkylsulphonyl group, an acyl group, a carboxylate group or a carboxamide group; and **n** is 0, 1 or 2.

**2.** Agent according to Claim 1, **characterized in that** the pyrazolone dye of the formula (I) is chosen from compounds of the formula (I) where
**R1** is a straight-chain or branched C1- to C4-alkyl group, a hydroxyethyl group, a dihydroxypropyl group, a methoxyethyl group, a C1- to C4-sulphoalkyl group, a phenyl radical, a phenyl radical substituted by one or more halogen atoms, a phenyl radical substituted by one or two sulphonic acid groups or a phenyl radical substituted by one or two carboxylic acid groups;
**R2** is hydrogen, a methyl group, a carboxylic acid group, a carboxylic acid group esterified with a straight-chain C1- to C4-alcohol, or a carboxamide group;
**Y** is a donor-substituted phenyl radical, an aromatic five-membered heterocyclic ring or a benzo-fused aromatic five-membered heterocyclic ring; and
**n** is 0, 1 or 2.

**3.** Agent according to Claim 1 or 2, **characterized in that** the physiologically compatible salt is chosen from pyridinium salts, tetraalkylphosphonium salts, tetraarylphosphonium salts, ammonium salts, triethylammonium salts, sodium salts, potassium salts, N-methylmorpholinium salts, monoethanolammonium salts, diethanolammonium salts and triethanolammonium salts.

**4.** Agent according to one of Claims 1 to 3, **characterized in that** the polymethine dye of the formula (I) is chosen

from 4-(4-(bis(2-hydroxyethyl)amino)benzylidene)-2-(2-hydroxyethyl)-5-methyl-2,4-dihydropyrazol-3-one; 2-(2-hydroxyethyl)-5-methyl-4-thiophen-2-ylmethylene-2,4-dihydropyrazol-2-one; 2-(2-hydroxyethyl)-5-methyl-4-thiophen-3-ylmethylene-2,4-dihydropyrazol-3-one; 4-(4-hydroxybenzylidene)-2-(2-hydroxyethyl)-5-methyl-2,4-dihydropyrazol-3-one; 4-(3-(4-dimethylaminophenyl)allylidene)-2-(2-hydroxyethyl)-5-methyl-2,4-dihydropyrazol-3-one; 4-(4-(1H-indol-3-ylmethylene)-3-methyl-5-oxo-4,5-dihydropyrazol-1-yl)benzenesulphonic acid; triethylammonium 4-(4-(1H-indol-3-ylmethylene)-3-methyl-5-oxo-4,5-dihydropyrazol-1-yl)-benzenesulphonate; 4-(4-dimethylaminobenzylene)-5-oxo-1-(4-sulphophenyl)-4,5-dihydro-1H-pyrazole-3-carboxylic acid; ammonium 4-(4-dimethylaminobenzylidene)-5-oxo-1-(4-sulphophenyl)-4,5-dihydro-1H-pyrazole-3-carboxylic acid; 4-(4-(4-dimethylaminobenzylidene)-3-methyl-5-oxo-4,5-dihydropyrazol-1-yl)benzenesulphonic acid; ammonium 4-(4-(4-dimethylaminobenzylidene)-3-methyl-5-oxo-4,5-dihydropyrazol-1-yl)benzenesulphonate; 4-chloro-3-(4-(4-dimethylaminobenzylidene)-3-methyl-5-oxo-4,5-dihydropyrazol-1-yl)benzenesulphonic acid; ammonium 4-chloro-3-(4-(4-dimethylaminobenzylidene)-3-methyl-5-oxo-4,5-dihydropyrazol-1-yl)benzenesulphonate; 4-(4-(4-hydroxy-3-methoxybenzylidene)-3-methyl-5-oxo-4,5-dihydropyrazol-1-yl)benzenesulphonic acid; 4-(4-(4-methoxybenzylidene)-3-methyl-5-oxo-4,5-dihydropyrazol-1-yl)benzenesulphonic acid; 4-(4-(2,4-dimethoxybenzylidene)-3-methyl-5-oxo-4,5-dihydropyrazol-1-yl)benzenesulphonic acid; ammonium 4-(4-(2,4-dimethoxybenzylidene)-3-methyl-5-oxo-4,5-dihydropyrazol-1-yl)benzenesulphonate; 4-(3-methyl-5-oxo-4-thiophen-2-ylmethylene-4,5-dihydropyrazol-1-yl)benzenesulphonic acid; ammonium 4-(3-methyl-5-oxo-4-thiophen-2-ylmethylene-4,5-dihydropyrazol-1-yl) benzenesulphonic acid; 4-(3-methyl-4-(1-methyl-1,2,3,4-tetrahydroquinolin-6-ylmethylene)-5-oxo-4,5-dihydropyrazol-1-yl)benzenesulphonic acid; 4-(4-(1-(2-cyanoethyl)-1,2,3,4-tetrahydroquinolin-6-ylmethylene)-3-methyl-5-oxo-4,5-dihydropyrazol-1-yl)benzenesulphonic acid; 4-(4-(3-(4-dimethylaminophenyl)allylidene)-3-methyl-5-oxo-4,5-dihydropyrazol-1-yl)benzenesulphonic acid; ammonium 4-(4-(3-(4-dimethylaminophenyl)allylidene)-3-methyl-5-oxo-4,5-dihydropyrazol-1-yl)benzenesulphonate; 4-(3-(4-dimethylaminophenyl)allylidene)-5-oxo-1-(4-sulphophenyl)-4,5-dihydro-1H-pyrazole-3-carboxylic acid; ammonium 4-(3-(4-dimethylaminophenyl)allylidene)-5-oxo-1-(4-sulphophenyl)-4,5-dihydro-1H-pyrazole-3-carboxylic acid; 3-(5-hydroxy-3-methyl-4-(2,4,6-trioxotetrahydropyrimidin-5-ylidenemethyl)pyrazol-1-yl)benzenesulphonic acid; 5-(1-(2,5-dichlorophenyl)-5-hydroxy-3-methyl-1H-pyrazol-4-ylmethylene)-2-thioxodihydropyrimidine-4,6-dione; 4-(4-(2,2-dimethyl-4,6-dioxo[1,3]dioxan-5-ylidenemethyl)-5-hydroxy-3-methylpyrazol-1-yl)benzenesulphonic acid; 4-(4-(5-(4,6-dioxo-1,3-dipropyl-2-thioxotetrahydropyrimidin-5-ylidene)penta-1,3-dienyl)-5-hydroxy-3-methylpyrazol-1-yl)benzenesulphonic acid; 2-(4-(4,4-bisethoxycarbonylbuta-1,3-dienyl)-5-hydroxy-3-methylpyrazol-1-yl)terephthalic acid; 1-(4-carboxyphenyl)-4-(4,4-dicarbamoylbuta-1,3-dienyl)-5-hydroxy-1H-pyrazole-3-carboxylic acid; 2-(4-(2,2-bisethanesulphonylvinyl)-5-hydroxy-3-methylpyrazol-1-yl)terephthalic acid; 2-(3-methyl-5-oxo-1-phenyl-1,5-dihydropyrazol-4-ylidenemethyl)benzenesulphonic acid and sodium 2-(3-methyl-5-oxo-1-phenyl-1,5-dihydropyrazol-4-ylidenemethyl) benzenesulphonate.

5. Agent according to one of Claims 1 to 4, **characterized in that** it comprises from 0.01 to 5 per cent by weight of the polymethine dye of the formula (I).

6. Agent according to one of Claims 1 to 5, **characterized in that** it has a pH of from 2 to 11.

7. Agent according to one of Claims 1 to 6, **characterized in that** it comprises a carrier.

8. Agent according to one of Claims 1 to 7, **characterized in that** it is a hair colorant.

9. Agent according to one of Claims 1 to 8, **characterized in that** it additionally comprises natural or synthetic polymers or modified polymers of natural origin.

10. Method for temporarily colouring hair, **characterized in that** 50 to 150 grams of an agent according to one of claims 1 to 8 are applied to the hair and, after a contact time of from 10 to 45 minutes at 20 to 50°C, the hair is rinsed with water, optionally washed with a shampoo and/or after-rinsed with an aqueous solution of weak organic acid, such as, for example, citric acid or tartaric acid, and is then dried, and the coloured hair is decoloured again at a later time using a reducing agent or oxidizing agent.

11. Method for temporarily colouring hair, **characterized in that** the hair is wetted using an agent according to Claim 9, styled and then dried, and the coloured hair is decoloured again at a later time using a reducing agent or oxidizing agent.

12. Method according to Claim 10 or 11, **characterized in that** the reducing agents used are alkali metal sulphites, alkali metal hydrogensulphites, alkali metal hyposulphites, alkali metal pyrosulphites, ammonium sulphite or ammonium hydrogensulphite.

**13.** Method according to Claim 10 or 11, **characterized in that** the reducing agent used is a combination of a) alkali metal sulphites, alkali metal hydrogensulphites, alkali metal pyrosulphites, ammonium sulphite or ammonium hydrogensulphite and b) reductones and/or thiols.

**14.** Method according to Claim 10 or 11, **characterized in that** the oxidizing agents used are bleaching powders comprising 5 to 50 per cent by weight of ammonium persulphate and/or alkali metal persulphates, alone or in combination with a hydrogen peroxide solution.

## Revendications

**1.** Composition pour la teinture de fibres de kératine, **caractérisée en ce qu'**elle contient au moins un colorant pyrazolone de formule générale (I) ou un sel physiologiquement acceptable d'un tel composé,

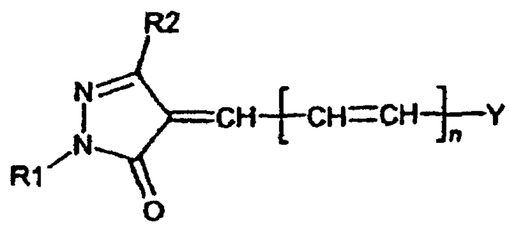

(I)

formule dans laquelle **R1** représente un atome d'hydrogène, un groupe alkyle en $C_1$-$C_8$ à chaîne droite ou ramifiée, un groupe hydroxyéthyle, un groupe dihydroxypropyle, un groupe méthoxyéthyle, un groupe carboxyéthyle, un groupe sulfoalkyle en $C_1$-$C_4$, un radical phényle, un radical phényle substitué par un ou plusieurs atomes d'halogène, un radical phényle substitué par un ou deux groupes sulfo, un radical phényle substitué par un ou deux groupes carboxy, un radical phényle substitué par un ou plusieurs groupes alkyle en $C_1$-$C_8$ ramifiés ou non ramifiés, un radical phényle substitué par un ou plusieurs groupes alcoxy en $C_1$-$C_8$ ramifiés ou non ramifiés, un radical benzyle, un radical benzyle substitué par un ou plusieurs atomes d'halogène, un radical benzyle substitué par un groupe alkyle en $C_1$-$C_4$, un radical benzyle substitué un groupe hydroxy, un radical benzyle substitué par un groupe méthoxy, un radical benzyle substitué par un groupe carboxy, un radical benzyle substitué par un groupe nitro, un radical benzyle substitué groupe amino, ou un hétérocycle à cinq ou six chaînons, saturé ou insaturé ;

**R2** représente un atome d'hydrogène, un groupe alkyle en $C_1$-$C_6$ ramifié ou non ramifié, un radical phényle, un groupe amino, un groupe amino acylé ou sulfonylé, un groupe acétyle, un groupe méthoxy, un groupe carboxy, un groupe carboxy estérifié par un alcool en $C_1$-$C_8$ à chaîne droite ou ramifiée ou par un éther monométhylique d'éthylèneglycol ou par un éther monoéthylique d'éthylèneglycol, un groupe carboxamido, un groupe carboxanilido ou un groupe 2-amino-2-oxyéthyle ;

**Y** représente un cycle aromatique carbocyclique ou hétérocyclique à cinq ou six chaînons, ou un cycle aromatique carbocyclique ou hétérocyclique à cinq ou six chaînons, soudé à un noyau benzénique, ou Y représente un radical de formule générale (II) ou (III),

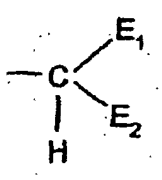

(II),

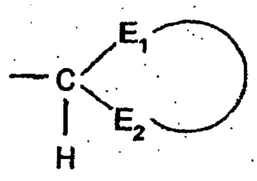

(III),

formules dans lesquelles **E1** et **E2** représentent, indépendamment l'un de l'autre, un groupe nitrile, un groupe alkylsulfonyle, un groupe acyle, un groupe ester d'acide carboxylique ou un groupe carbamoyle ; et n à la valeur

0, 1 ou 2.

2. Composition selon la revendication 1, **caractérisée en ce que** le colorant pyrazolone de formule (I) est choisi parmi des composés de formule (I) dans lesquels

**R1** représente un groupe alkyle en $C_1$-$C_4$ à chaîne droite ou ramifiée, un groupe hydroxyéthyle, un groupe dihydroxypropyle, un groupe méthoxyéthyle, un groupe sulfoalkyle en $C_1$-$C_4$, un radical phényle, un radical phényle substitué par un ou plusieurs atomes d'halogène, un radical phényle substitué par un ou deux groupes sulfo ou un radical phényle substitué par un ou deux groupes carboxy ;
**R2** représente un atome d'hydrogène, un groupe méthyle, un groupe carboxy, un groupe carboxy estérifié par un alcool en $C_1$-$C_4$ à chaîne droite ou un groupe carboxamido ;
**Y** représente un radical phényle substitué par un donneur, un cycle aromatique hétérocyclique à cinq chaînons, ou un cycle aromatique hétérocyclique à cinq chaînons, soudé à un noyau benzénique ; et
n à la valeur 0, 1 ou 2.

3. Composition selon la revendication 1 ou 2, **caractérisée en ce que** le sel physiologiquement acceptable est choisi parmi les sels de pyridinium, les sels de tétraalkylphosphonium, les sels de tétraarylphosphonium, les sels d'ammonium, les sels de triéthylammonium, les sels de sodium, les sels de potassium, les sels de N-méthylmorpholinium, les sels de monoéthanolammonium, les sels de diéthanolammonium et les sels de triéthanolammonium.

4. Composition selon l'une quelconque des revendications 1 à 3, **caractérisée en ce que** le colorant polyméthine de formule (I) est choisi parmi la 4-(4-(bis-(2-hydroxyéthyl)amino)-benzylidène)-2-(2-hydroxyéthyl)-5-méthyl-2,4-dihydropyrazol-3-one, la 2-(2-hydroxyéthyl)-5-méthyl-4-thiophén-2-ylméthylène-2,4-dihydropyrazol-3-one, la 2-(2-hydroxyéthyl)-5-méthyl-4-thiophén-3-ylméthylène-2,4-dihydropyrazol-3-one, la 4-(4-hydroxybenzylidène)-2-(2-hydroxyéthyl)-5-méthyl-2,4-dihydropyrazol-3-one, la 4-(3-(4-diméthylamino-phényl)-allylidène)-2-(2-hydroxyéthyl)-5-méthyl-2,4-dihydropyrazol-3-one, l'acide 4-(4-(1H-indol-3-yl-méthylène)-3-méthyl-5-oxo-4,5-dihydro-pyrazol-1-yl)-benzènesulfonique, le 4-(4-(1H-indol-3-yl-méthylène)-3-méthyl-5-oxo-4,5-dihydro-pyrazol-1-yl)-benzènesulfonate de triéthylammonium, l'acide 4-(4-diméthylamino-benzylidène)-5-oxo-1-(4-sulfophényl)-4,5-dihydro-1H-pyrazol-3-carboxylique, le 4-(4-diméthylamino-benzylidène)-5-oxo-1-(4-sulfophényl)-4,5-dihydro-1H-pyrazole-3-carboxylate d'ammonium, l'acide 4-(4-(4-diméthylamino-benzylidène)-3-méthyl-5-oxo-4,5-dihydro-pyrazol-1-yl)-benzènesulfonique, le 4-(4-(4-diméthylaminobenzylidène)-3-méthyl-5-oxo-4,5-dihydro-pyrazol-1-yl) benzènesulfonate d'ammonium, l'acide 4-chloro-3-(4-(4-diméthylamino-benzylidène)-3-méthyl-5-oxo-4,5-dihydro-pyrazol-1-yl)benzènesulfonique, le 4-chloro-3-(4-(4-diméthylamino-benzylidène)-3-méthyl-5-oxo-4,5-dihydro-pyrazol-1-yl)benzènesulfonate d'ammonium, l'acide 4-(4-(4-hydroxy-3-méthoxy-benzylidène)-3-méthyl-5-oxo-4,5-dihydropyrazol-1-yl)benzènesulfonique, l'acide 4-(4-(4-méthoxy-benzylidène)-3-méthyl-5-oxo-4,5-dihydropyrazol-1-yl)benzènesulfonique, l'acide 4-(4-(2,4-diméthoxy-benzylidène)-3-méthyl-5-oxo-4,5-dihydropyrazol-1-yl)benzènesulfonique, le 4-(4-(2,4-diméthoxy-benzylidène)-3-méthyl-5-oxo-4,5-dihydropyrazol-1-yl)benzènesulfonate d'ammonium, l'acide 4-(3-méthyl-5-oxo-4-thiophén-2-ylméthylène-4,5-dihydro-pyrazol-1-yl)benzènesulfonique, le 4-(3-méthyl-5-oxo-4-thiophén-2-ylméthylène-4,5-dihydropyrazol-1-yl)benzènesulfonate d'ammonium, l'acide 4-(3-méthyl-4-(1-méthyl-1,2,3,4-tétrahydroquinolin-6-ylméthylène)-5-oxo-4,5-dihydro-pyrazol-1-yl)benzènesulfonique, l'acide 4-(4-(1-(2-cyanoéthyl)-1,2,3,4-tétrahydro-quinolin-6-ylméthylène)-3-méthyl-5-oxo-4,5-dihydro-pyrazol-1-yl)benzènesulfonique, l'acide 4-(4-(3-(4-diméthylaminophényl)-allylidène)-3-méthyl-5-oxo-4,5-dihydropyrazol-1-yl)benzènesulfonique, le 4-(4-(3-(4-diméthylamino-phényl)-allylidène)-3-méthyl-5-oxo-4,5-dihydro-pyrazol-1-yl)benzènesulfonate d'ammonium, l'acide 4-(3-(4-diméthylamino-phényl)-allylidène)-5-oxo-1-(4-sulfophényl)-4,5-dihydro-1H-pyrazole-3-carboxylique, le 4-(3-(4-diméthylamino-phényl)-allylidène)-5-oxo-1-(4-sulfophényl)-4,5-dihydro-1H-pyrazole-3-carboxylate d'ammonium, l'acide 3-(5-hydroxy-3-méthyl-4-(2,4,6-trioxotétrahydropyrimidin-5-ylidèneméthyl)-pyrazol-1-yl)benzènesulfonique, la 5-(1-(2,5-dichlorophényl)-5-hydroxy-3-méthyl-1H-pyrazol-4-yl-méthylène)-2-thioxo-dihydro-pyrimidine-4,6-dione, l'acide 4-(4-(2,2-diméthyl-4,6-dioxo-[1,3]dioxan-5-ylidèneméthyl)-5-hydroxy-3-méthyl-pyrazol-1-yl)benzènesulfonique, l'acide 4-(4-(5-(4,6-dioxo-1,3-dipropyl-2-thioxo-tétrahydropyrimidin-5-yl-idène)-penta-1,3-diényl)-5-hydroxy-3-méthyl-pyrazol-1-yl)-benzènesulfonique, l'acide 2-(4-(4,4-bis-éthoxy-carbonyl-buta-1,3-diényl)-5-hydroxy-3-méthyl-pyrazol-1-yl)-téréphtalique, l'acide 1-(4-carboxyphényl)-4-(4,4-dicarbamoyl-buta-1,3-diényl)-5-hydroxy-1H-pyrazole-3-carboxylique, l'acide 2-(4-(2,2-bis-éthanesulfonyl-vinyl)-5-hydroxy-3-méthyl-pyrazol-1-yl-téréphtalique, l'acide 2-(3-méthyl-5-oxo-1-phényl-1,5-dihydropyrazol-4-ylidèneméthyl)benzènesulfonique et le 2-(3-méthyl-5-oxo-1-phényl-1,5-dihydropyrazol-4-ylidèneméthyl)benzènesulfonate de sodium.

5. Composition selon l'une quelconque des revendications 1 à 4, **caractérisée en ce qu'**elle contient de 0,01 à 5 % en poids du colorant polyméthine de formule (I).

6. Composition selon l'une quelconque des revendications 1 à 5, **caractérisé en ce qu'**elle présente un pH de 2 à 11.

7. Composition selon l'une quelconque des revendications 1 à 6, **caractérisé en ce qu'**elle contient un véhicule.

8. Composition selon l'une quelconque des revendications 1 à 7, **caractérisé en ce qu'**elle est une composition de teinture pour cheveux.

9. Composition selon l'une quelconque des revendications 1 à 8, **caractérisé en ce qu'**elle contient en outre des polymères naturels ou synthétiques ou des polymères d'origine naturelle, modifiés.

10. Procédé pour la teinture temporaire des cheveux, **caractérisé en ce qu'**on applique sur les cheveux de 50 à 150 grammes d'une composition selon l'une quelconque des revendications 1 à 8, et, après un temps d'action de 10 à 45 minutes à 20-50°C, on rince les cheveux à l'eau, éventuellement on les lave avec un shampooing et/ou on les soumet à un après-lavage avec la solution aqueuse d'un acide organique faible, comme par exemple l'acide citrique ou l'acide tartrique, et ensuite on les sèche, et les cheveux teints sont de nouveau décolorés ultérieurement à l'aide d'un réducteur
    ou d'un oxydant.

11. Procédé pour la teinture temporaire des cheveux, **caractérisé en ce que** les cheveux sont humectés avec une composition selon la revendication 9, mis en plis et ensuite séchés, et les cheveux teints sont de nouveau décolorés ultérieurement à l'aide d'un réducteur ou d'un oxydant.

12. Procédé selon la revendication 10 ou 11, **caractérisé en ce qu'**on utilise comme réducteur des sulfites de métaux alcalins, des hydrogénosulfites de métaux alcalins, des hyposulfites de métaux alcalins, des pyrosulfites de métaux alcalins, du sulfite d'ammonium ou de l'hydrogénosulfite d'ammonium.

13. Procédé selon la revendication 10 ou 11, **caractérisé en ce qu'**on utilise comme réducteur une association de a) sulfites de métaux alcalins, hydrogénosulfites de métaux alcalins, pyrosulfites de métaux alcalins, sulfite d'ammonium ou hydrogénosulfite d'ammonium et b) réductones ou thiols.

14. Procédé selon la revendication 10 ou 11, **caractérisé en ce qu'**on utilise comme oxydant de 5 à 50 % en poids d'une poudre de teinture en blond contenant du persulfate d'ammonium et/ou des persulfates de métaux alcalins, seule ou en association avec une solution de peroxyde d'hydrogène.